# EUROPEAN PATENT APPLICATION

(11) **EP 3 372 590 A1**
(43) Date of publication of application: **12.09.2018**
(21) Application number: 16861532.6
(22) Date of filing: 01.11.2016
(51) Int. Cl.: C07D 239/60, C07D 239/54, C07D 309/38, C07D 213/69, C07C 49/825, C07C 39/08, C07D 239/56, C07D 239/48, C07D 251/20, C07D 239/545, A61K 31/505

(54) **COMPOUNDS HAVING AGONISTIC EFFECT FOR GPR84, PREPARATION METHOD FOR COMPOUNDS AND USE OF COMPOUNDS**

(30) Priority: 04.11.2015 CN 201510745971
(71) Applicant: Shanghai Institute of Materia Medica, Chinese Academy of Sciences, Shanghai 201203 (CN)
(72) Inventor: NAN, Fajun, Pudong Shanghai 201203 (CN); XIE, Xin, Pudong Shanghai 201203 (CN); LIU, Yang, Pudong Shanghai 201203 (CN); ZHANG, Qing, Pudong Shanghai 201203 (CN); CHEN, Linhai, Pudong Shanghai 201203 (CN); YANG, Hui, Pudong Shanghai 201203 (CN)
(74) Representative: Grünecker Patent- und Rechtsanwälte PartG mbB
(86) International application number: PCT/CN2016/104165
(87) International publication number: WO 2017/076264

(57) **Abstract**

The present invention relates to a class of compounds represented by the formula **I**, or pharmaceutically acceptable salts thereof, methods for their preparation, and application as small molecule tools that function as GPR84 agonists, and their use in preparing a medicament for the treatment of septicemia.

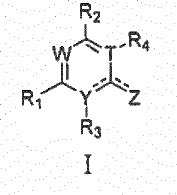

## Description

### TECHNICAL FIELD

The present invention relates to a class of compounds having an agonistic effect against GPR84 represented by the formula **I,** methods for their preparation, and their use in preparing a medicament for the treatment of septicemia.

### BACKGROUND

G protein-coupled receptors (GPCRs) are a large family of membrane protein receptors, with 367 human gene encoding related proteins, which are almost involved in the regulation of all physiological functions of the cell. Currently, there are many known GPCRs ligands such as gases, hormones, neurotransmitters, and chemokines. In addition, it has been found that many free fatty acids (FFAs) are endogenous ligands of GPCRs. The FFAs mainly bind to free fatty acid receptors (FFARs) so as to activate a series of downstream cell pathways, thereby regulating the body. Studies show that FFAs play an important role in glucose homeostasis, lipid formation, and immune responses (Tomo Yonezawa et al. Free Fatty Acids-Sensing G Protein-Coupled Receptors in Drug Targeting and Therapeutics. Current Medicinal Chemistry 2013, 20 (30), 3855-3871).

GPR84 (G protein-coupled receptor 84) is also a class of membrane protein receptors, consisting of seven transmembrane alpha helices composed of 25-35 contiguous amino acids. It is the recently identified one type of rhodopsin-like receptor. GPR84 was first cloned by Wittenberger using the Expressed Sequence Tag (EST) method. Human's GPR84 is located on chromosome 12, while mouse's GPR84 is located on chromosome 15. GPR84 is expressed mainly in bone marrow, peripheral blood leukocytes (including neutrophils, eosinophils, and basophils). (Timo Wittenberger et al. An Expressed Sequence Tag (EST) Data Mining Strategy Succeeding in the Discovery of New G-Protein Coupled Receptors. Journal of Molecular Biology 2001, 303 (3), 799-813). Medium-chain FFAs, through their direct actions on GPR84, may remarkably affect interleukin-12 p40 subunits (IL-12 p40) from RAW264.7 murine macrophage-like cells at the present of LPS. The IL-12 p40 plays a pivotal in promoting cell-mediated immunity, it can protect maintaining T helper1 (Th1) responses and inhibit T helper2 (Th2) responses. (Wang, J et al, Medium-chain fatty acids as ligands for orphan G protein-coupled receptor GPR84. The Journal of biological chemistry 2006, 281 (45), 34457-64.) Therefore, GPR84 may affect Th1/Th2 balance and may play a role in the autoimmune diseases and inflammatory diseases, such as multiple sclerosis, inflammatory bowel disease, and arthritis. In addition, the occurrence of metabolic diseases such as obesity and diabetes is closely related to chronic inflammation. When macrophages infiltrate adipose tissue, the inflammatory response can be promoted by secreting cytokines, and GPR84 expression will increase in adipocytes. The results showed that GPR84 was also involved in the regulation between fatty acid metabolism and the immune system (Perseghin, G.; Petersen, K.; Shulman, G. I., Cellular mechanism of insulin resistance: potential links with inflammation. International journal of obesity and related metabolic disorders: Journal of the International Association for the Study of Obesity 2003, 27 Suppl 3, S6-11.).

In 2006, the Ling group reported that GPR84 could not be activated by either long-chain free fatty acids (LCFA) or short-chain free fatty acids (SCFA), but could be activated by medium-chain free fatty acids (MCFA). The most potent agonists include the capric acid (C10:0), undecanoic acid (C11:0), and lauric acid (C12:0) with potencies of 4 µM, 8 µM, and 9 µM, respectively (CHO-GPR84 cells, cAMP assay). In addition, they also reported a compound named diindolylmethane (DIM) having a better activity (EC₅₀ = 0.5 µM in [35S]-GTPyS binding assay, and EC₅₀ = 0.7 µM in cAMP assay). Suzuki et al. reported that 2- or 3-hydroxy Medium-chain fatty acids (2-OH-C10, 3-OH-C10, 2-OH-C12, 3-OH-C12) also have some GPR84 agonist activities, and 6-n-octylaminouracil (6-OAU) exhibits a profile of activity as an agonist of GPR84 during high throughput screening (EC₅₀=0.661µM)(Suzuki, M.et at. Medium-chain fatty acid-sensing receptor, GPR84, is a proinflammatory receptor. The Journal of biological chemistry 2013, 288 (15), 10684-91).

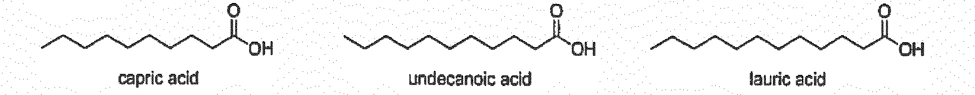

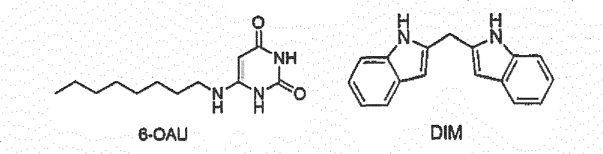

Since the normal physiological expression of GPR84 is low, and high expression can be observed only under certain stimuli, meanwhile, the expression level of GPR84 has a tight relationship with inflammation, GPR84 is a very good target for the treatment of inflammation-related diseases. However, it is not clear about the specific mechanism currently. There are no good GPR84 agonists at present, which hinder the further mechanism study of GPR84. Therefore, it is highly desirable to develop the agonists of GPR84.

This invention relates to a series of small molecule compounds, which have good agonistic activities to GPR84, and have a dramatic improvement in the activity compared with reported GPR84 agonists. Thereby a better small molecule tool is provided for studying the mechanism of action of GPR84. Additionally, new ways will be developed for septicemia drugs exploring.

### SUMMARY OF THE INVENTION

One object of the present invention is to design and synthesize a class of compounds, which can be used as an agonist of GPR84, thereby providing a good tool for the study of the action mechanism of GPR84, as well as exploring a new way for developing drugs for the treatment of septicemia.

Another object of the present invention is to provide a method for preparing the above-described compound.

Another object of the present invention is to provide a pharmaceutical composition comprising one or more above-described compound or a pharmaceutically acceptable salt thereof and one or more pharmaceutically acceptable carriers.

A further object of the present invention is to provide the use of the above-described compound.

The present invention provides a compound represented by formula **I** or a pharmaceutically acceptable salt thereof, 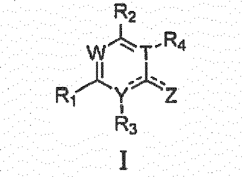 wherein, R₁ is R₁ₐ, R_{1b} or R_{1c}; 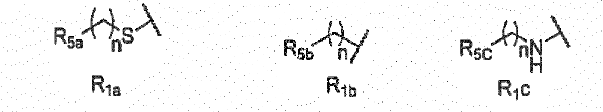
each of R₅ₐ, R_{5b} and R_{5c} is independently methyl, isopropyl, C2-C9 alkenyl, C2-C4 alkynyl, 3-6 membered cycloalkyl, cyano, hydroxy, unsubstituted phenyl, phenyl substituted with C1-C4 alkyl, phenyl substituted with C1-C3 alkoxy, or fluorophenyl; preferably, each of R₅ₐ, R_{5b} and R_{5c} is independently methyl, isopropyl, C2-C9 alkenyl, ethynyl, 3-4 membered cycloalkyl, cyano, hydroxy, unsubstituted phenyl, phenyl substituted with C1-C4 alkyl, methoxyphenyl, or fluorophenyl;
subscript n is an integer selected from 0-16; preferably, subscript n is an integer selected from 0-9;
T, W and Y are each independently O, N or C; preferably, T, W and Y are each independently N or C;
R₂ is hydroxy, amino, trifluoromethyl, or C1-C3 alkyl; preferably, R₂ is hydroxy, amino, trifluoromethyl, or methyl;
R₃ is absent or is hydrogen, benzyl or C1-C6 alkyl; preferably, R₃ is absent or is hydrogen or benzyl;
R₄ is absent or is hydrogen or C1-C3 alkyl; preferably, R₄ is absent or is hydrogen or methyl;
Z is -OH, -NH₂, =O, =S or C1-C6 alkylcarbonyl.

In one embodiment, in formula **I,** R₁ is R₁ₐ, wherein R₅ₐ is methyl, isopropyl, 3-4 membered cycloalkyl, unsubstituted phenyl, cyano, hydroxy, phenyl substituted with C1-C4 alkyl, methoxyphenyl, or fluorophenyl;
R₂ is hydroxy;
subscript n is an integer selected from 0-14;
T, W and Y are each independently O, N or C;
R₃ is absent or is hydrogen, benzyl or C1-C3 alkyl;
R₄ is absent or is hydrogen or C1-C3 alkyl;
Z is -OH, -NH₂, =O, =S or C1-C6 alkylcarbonyl.

In one embodiment, the compound of the formula **I** has the structure of Formula **II**: 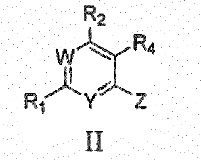
R₁ is R₁ₐ, R_{1b} or R_{1c};
R₂ is hydroxy, methyl, amino, or trifluoromethyl;
W and Y are each independently N;
R₄ is hydrogen or C1-C3 alkyl;
Z is -OH or -NH₂.

In one embodiment, the compound of the formula **I** has the structure of Formula **III:** 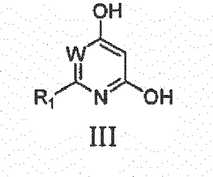 wherein,
R₁ is R₁ₐ, R_{1b} or R_{1c};
W is N or C.

The compound of the formula **I** is selected from:

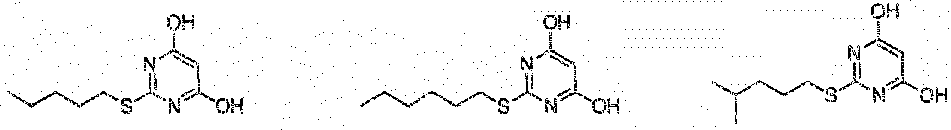

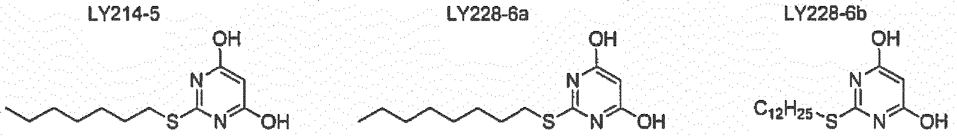

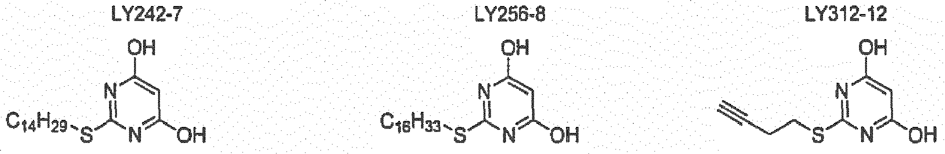

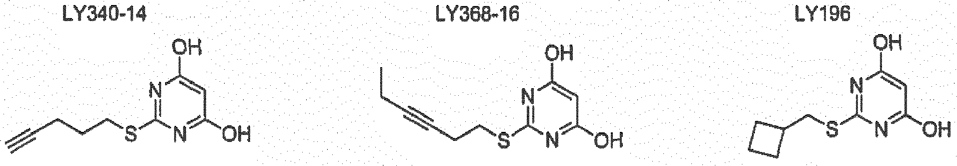

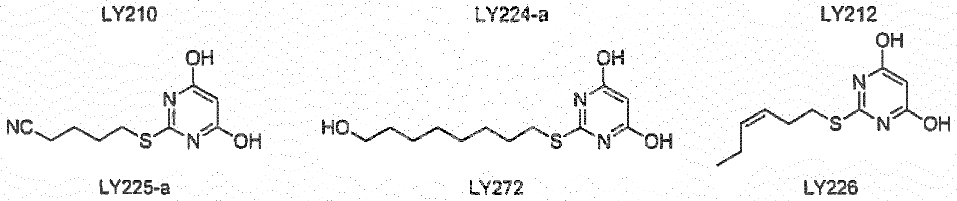

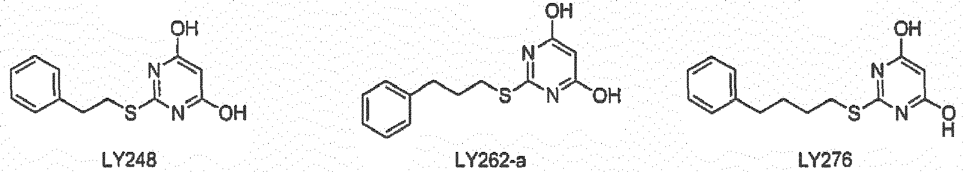

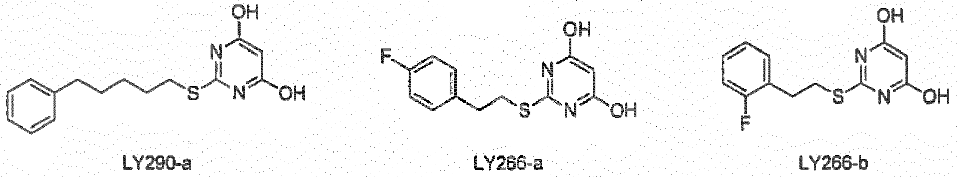

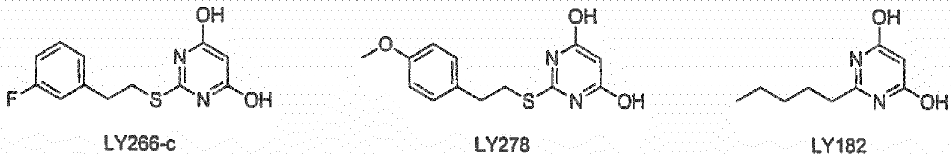

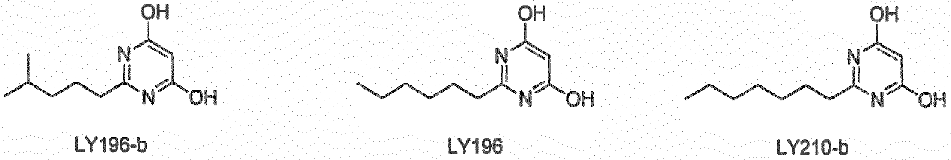

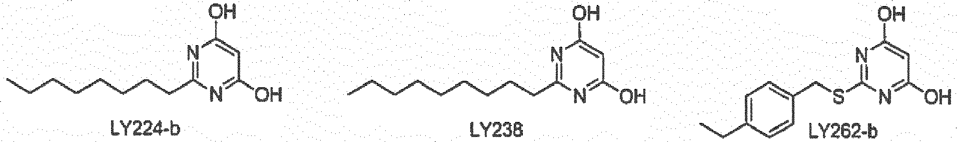

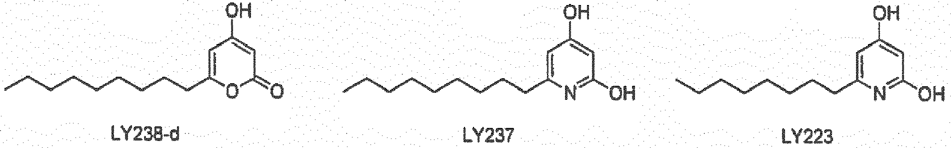

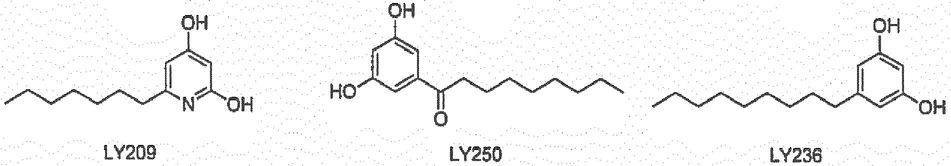

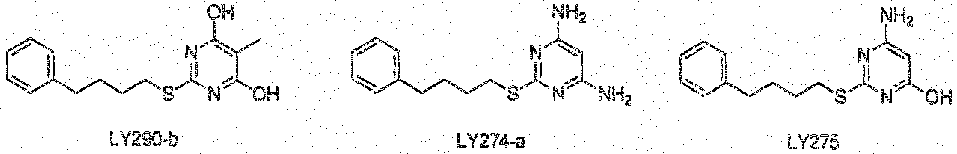

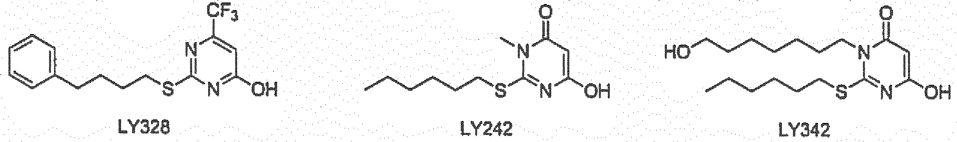

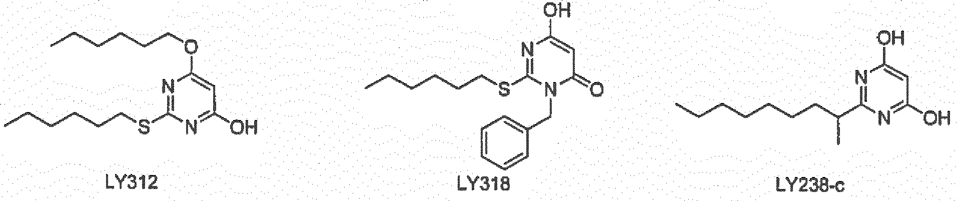

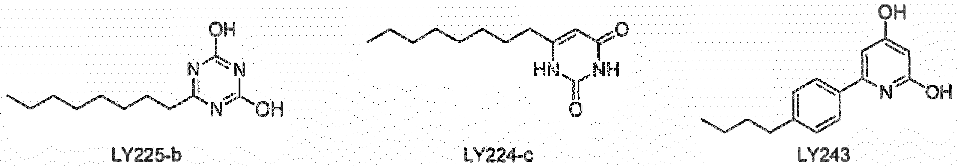

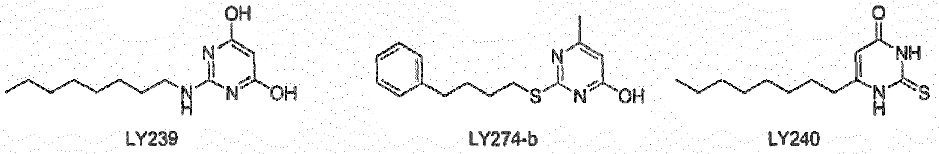

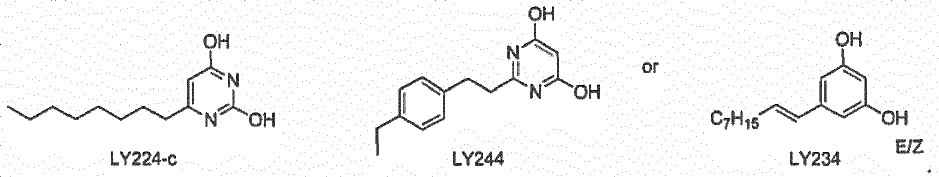

The compounds represented by formula **I** can be prepared by conventional methods, for example, as follows: 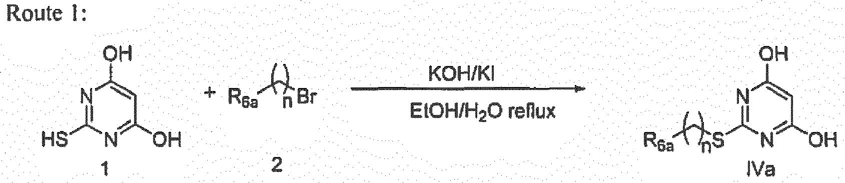
wherein, R₆ₐ is methyl, isopropyl, C2-C4 alkynyl, 3-6 membered cycloalkyl, cyano, hydroxy, unsubstituted phenyl, phenyl substituted with C1-C3 alkyl, phenyl substituted with C1-C3 alkoxy, or fluorophenyl;
subscript n is an integer selected from 1-16;
dissolving compound **1** in EtOH/H₂O (2:1), adding KOH and KI, then slowly adding the bromide **2** dropwise, heating at 80°C for 6 hours; thereafter, purifying with flash chromatography on silica gel to obtain product **IVa;** 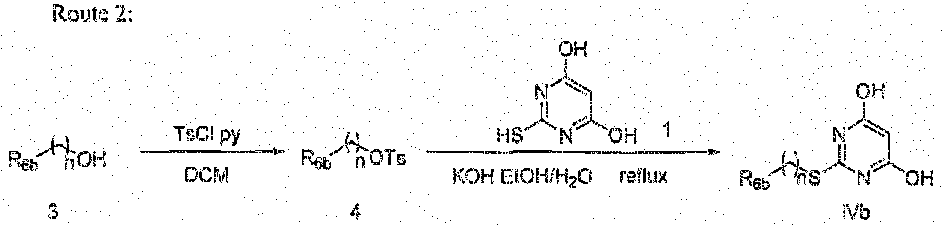
wherein, R_{6b} is C2-C9 alkenyl, C2-C4 alkynyl, 3-6 membered cycloalkyl, or phenyl substituted with C1-C3 alkyl;
subscript n is an integer selected from 1-16;
dissolving compound **3** and TsCl in anhydrous DCM, adding pyridine; maintaining the mixture at 25°C for 3 h; purifying with flash chromatography on silica gel to give compound 4; dissolving said compound **4** and compound **1** in EtOH/H₂O (2:1) and heating the solution to reflux for 6 h; after full completion, purifying using flash chromatography on silica gel to afford product IVb; 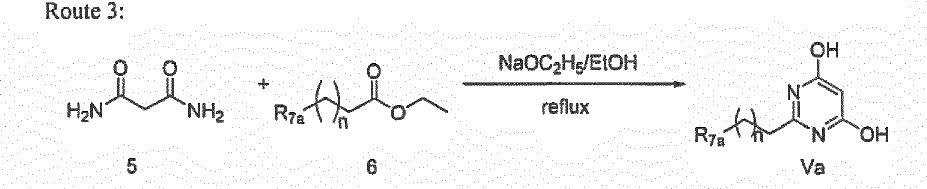
wherein, R₇ₐ is methyl, isopropyl, phenyl substituted with C1-C3 alkyl, methoxyphenyl, or fluorophenyl;
subscript n is an integer selected from 1-15;
adding compound **5** and compound **6** to a suspension of sodium in ethanol, heating at 80°C for 6 hours; concentrating the reaction mixture, followed by the addition of a small amount of water; adjusting the pH to acidity and forming a white precipitate, then filtering to afford the desired product; 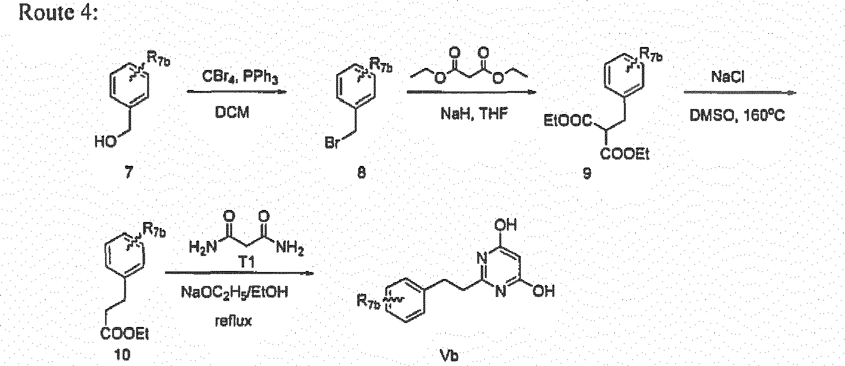
wherein, R_{7b} is methyl, ethyl, or isopropyl;
adding CBr₄ and PPh₃ to a solution of compound **7** in DCM; maintaining the reaction mixture at 25°C for 14h; After reaction completion, purifying by flash chromatography on silica gel to afford compound **8;** adding NaH to a solution of compound **8** in THF at -78°C, after 5 minutes, addition of diethyl malonate; gradually warming the reaction to 25°C. and stirring at 25°C for 6h; purifying with flash chromatography on silica gel to obtain compound **9;** stirring a solution of said compound **9** and NaCl in DMSO at 180°C for 3h; after reaction completion, purifying by flash chromatography on silica gel to afford compound **10;** adding compound **T1** and compound **10** to a suspension of sodium in ethanol; stirring the reaction mixture at 80°C for 6h; concentrating the reaction mixture, followed by the addition of a small amount of water; adjusting the pH to acidity and forming a white precipitate, then filtering to afford the desired product Vb; 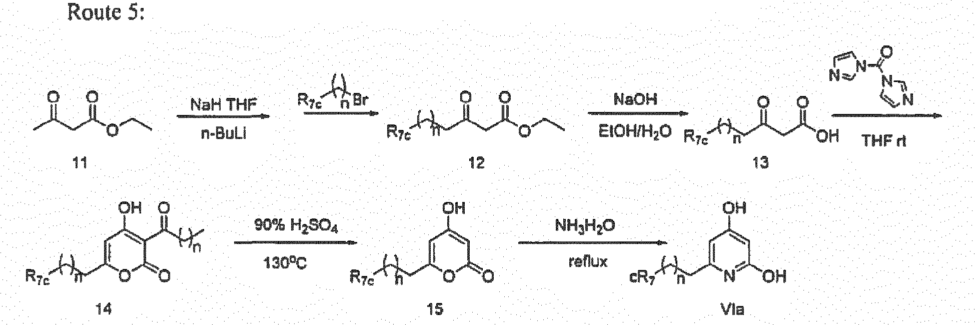
wherein, R_{7c} is methyl or isopropyl;
subscript n is an integer selected from 7-12;
adding NaH to a solution of compound **11** in THF at 0°C, after 5 minutes, adding *n*-BuLi; after stirring for 10 minutes, adding the bromide; stirring the reaction mixture at 0°C for 14 h; purifying by flash chromatography on silica gel to afford compound **12;** adding NaOH to a solution of compound **12** in EtOH/H₂O(2:1); then, maintaining the mixture at 25°C for 14 h; adjusting the pH to 4-5 with hydrochloric acid and forming a white precipitate, then filtering to afford the compound **13;** adding 1,1'-carbonyldiimidazole to a solution of compound **13** in THF and stirring the solution at 25°C for 14 h; purifying by flash chromatography on silica gel to afford compound **14;** heating a solution of compound **14** and 90% H₂SO₄ at 130 °C for 1h; purifying by flash chromatography on silica gel to afford compound **15**; heating a solution of compound **15** and 28% ammonium hydroxide at 100 °C for 14h; adjusting the pH to 4-5 using hydrochloric acid and forming a white precipitate, then filtering to obtain the compound **VIa;** 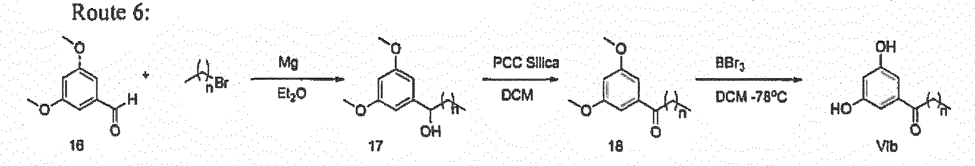
wherein, subscript n is an integer selected from 4-10;
adding the bromide dropwise to a suspension of the magnesium and ether, stirring the mixture for 1 h, then adding compound **16;** purifying by flash chromatography on silica gel to afford compound **17;**
adding PCC to a solution of compound **17** in DCM, then purifying by flash chromatography on silica gel to afford compound **18;**
adding BBr₃ to a solution of compound **18** in DCM at -78°C; gradually warming to 25°C and maintaining at 25°C for 14h; purifying crude compound **VIb** by flash chromatography on silica gel; 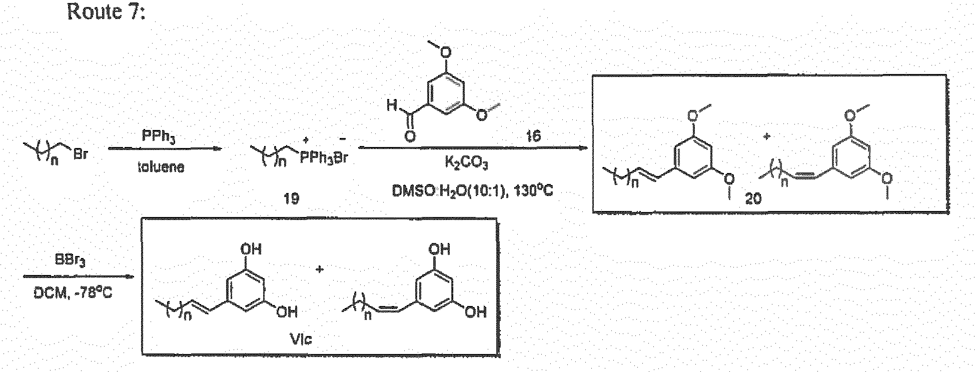
wherein, subscript n is an integer selected from 5-10;
adding triphenylphosphine to a solution of bromide in toluene; heating the solution at 120 °C for 14 h to obtain compound **19;** heating a mixture comprising the compound **19** and solvent DMSO/H₂O (10:1) at 130 °C for 3 h; then purifying by flash chromatography on silica gel to afford compound **20** (A mixture of Z and E configurations);
adding BBr₃ at -78°C to a solution of compound **20** in DCM, gradually warming to 25°C and stirring at 25°C for 14h, and then purifying by flash chromatography on silica gel to afford compound **VIc** (A mixture of Z and E configurations). 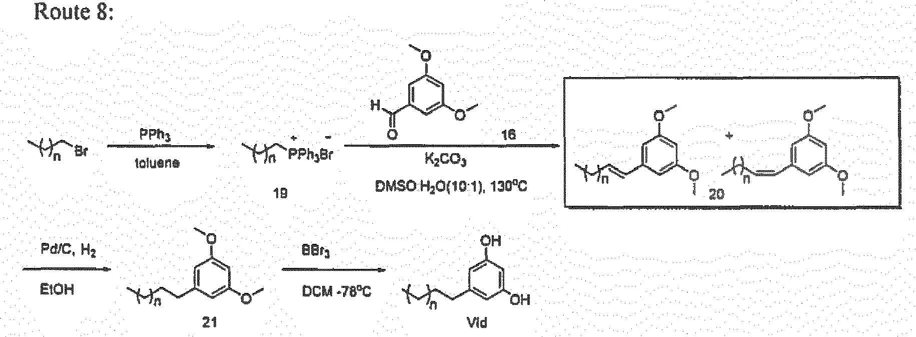
wherein, subscript n is an integer selected from 5-10;
using the similar synthetic method as in Route 7 to prepare compound **20;** then dissolving compound **20** (a mixture of Z and E configurations) in ethanol, adding Pd/C and stirring the reaction mixture in an atmosphere of H₂ for 16 h; purifying by flash chromatography on silica gel to afford compound **21;**
adding BBr₃ at -78°C to a solution of compound **21** in DCM, gradually warming to 25°C and maintaining at 25°C for 14h; purifying by flash chromatography on silica gel to afford compound **VId;** 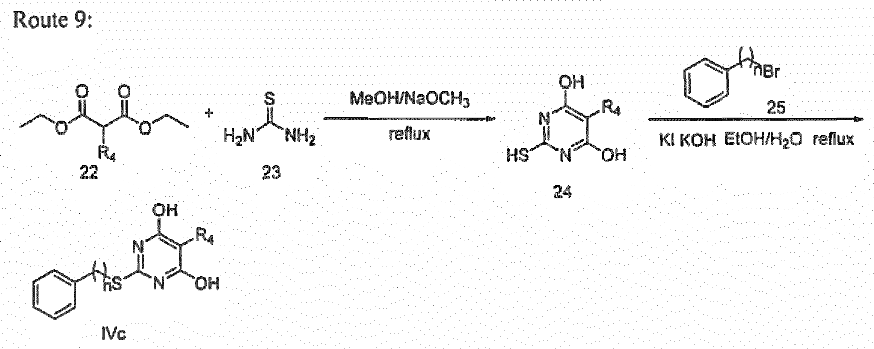
wherein, R₄ is hydrogen, or C1-C3 alkyl;
subscript n is an integer selected from 1-4;
adding compound **22** and compound **23** to a suspension of sodium in ethanol, and heating at 80°C for 16h; concentrating the reaction mixture, adding a small amount of water, and adjusting the pH to acidity; forming a white precipitate, then filtering to afford the compound **24;**
dissolving the compound **24** in EtOH/H₂O, then adding KI, KOH and bromide **25,** and heating at 80°C for 6h; followed by concentration and purification with flash chromatography on silica gel to afford compound **IVc;** 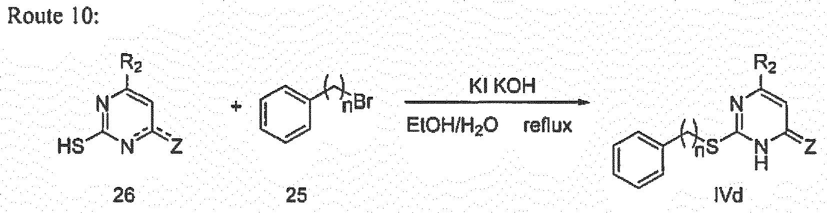
wherein, R₂ is amino, hydroxy, or methyl;
subscript n is an integer selected from 1-4;
Z is -OH, -NH₂, =O, =S or C1-C6 alkylcarbonyl;
adding compound **26,** compound **25,** KI and KOH to a suspension of sodium in ethanol, and heating at 80°C for 16h; concentrating the reaction mixture, adding a small amount of water, adjusting the pH to acidity; forming a white precipitate, then filtering to afford the compound **IVd;** 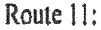 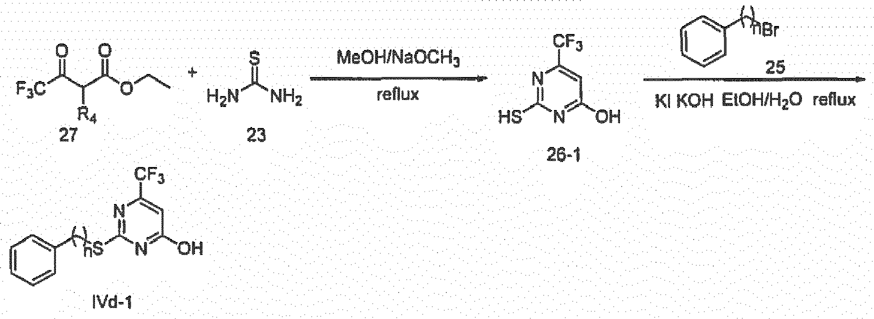
wherein, subscript n is an integer selected from 1-4;
R₄ is hydrogen, or methyl;
adding compound **27** and compound **23** to a suspension of sodium in ethanol, and heating at 80°C for 16h; concentrating the reaction mixture, adding a small amount of water, adjusting the pH to acidity; forming a white precipitate, then filtering to afford the compound **26-1;**
dissolving the compound **26-1** in EtOH/H₂O, then adding KI, KOH and bromide **25,** heating at 80°C for 6h; followed by concentration and purification with flash chromatography on silica gel to afford compound IVd-1; 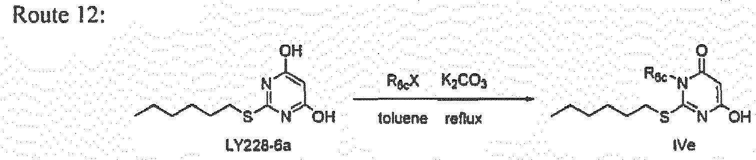
wherein,
R_{6c} is C1-C6 alkyl, or benzyl;
X is I, or Br;
adding K₂CO₃ to a solution of **LY228-6a** in toluene, cooling to 0°C, then, adding R_{6c}X; refluxing the reaction mixture for 12h; followed by concentration and purification with flash chromatography on silica gel to afford compound **IVe**; 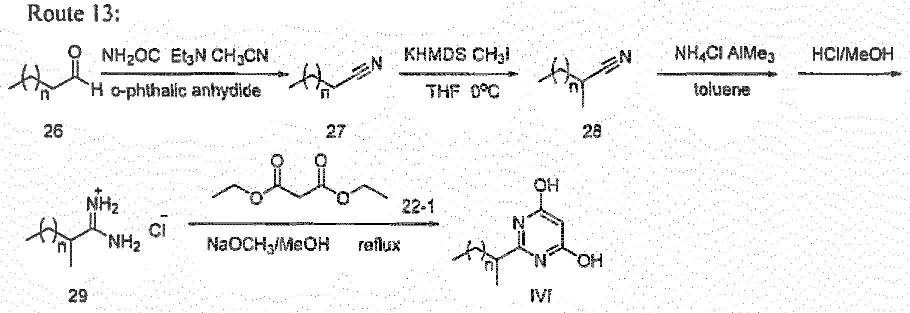
subscript n is an integer selected from 1-9;
Adding hydrazine monohydrochloride and triethylamine to a solution of compound **26** in acetonitrile at 0°C; warming to room temperature, and stirring for 30 minutes, then adding phthalic anhydride; refluxing the reaction mixture for 16h; concentrating the reaction mixture and adding DCM; filtering to remove the filter residue and washing with 5% Ammonium hydroxide solution; concentration of the organic phase, followed by dryness to obtain compound **27;**
adding KHMDS slowly to a solution of compound **27** in THF at 0°C; stirring for 5 minutes, adding iodomethane; monitoring the reaction with TLC; quenching, extraction, concentration, and purification with flash chromatography on silica gel to afford compound **28;**
adding trimethylaluminum slowly dropwise to a solution of NH₄Cl in anhydrous toluene at 0°C, under the N₂ atmosphere; adding a solution of compound **28** in toluene slowly dropwise to the reaction mixture until no methane gas emission; heating the reaction mixture to 80°C for 15 hours; and then cooling to room temperature, adding a small amount of silica gel, and stirring for 10 minutes; filtering resulting suspension, concentrating the filtrate; adding hydrochloric acid in methanol and stirring for 12h; concentration to obtain crude product **29;**
Adding said crude product **29** and compound **22-1** to a suspension of sodium in methanol; refluxing for 12h, and concentrating; adding a small amount of water until the solid dissolved; and then, adjusting the pH to acidity, crystallizing from the liquid medium, filtering the resulting suspension; and purifying the filter residue with flash chromatography on silica gel to afford compound **IVf**; 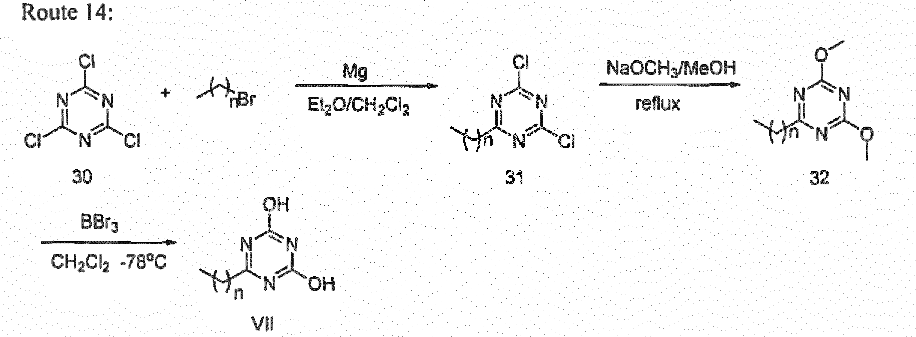
subscript n is an integer selected from 1-9;
adding the bromide dropwise to a suspension of the magnesium and ether, stirring the mixture for 1 h, then adding compound **30;** purifying by flash chromatography on silica gel to afford compound **31;**
dissolving said compound **31** in NaOCH₃/MeOH, heating to reflux for 16h to obtain compound **32;**
adding BBr₃ dropwise to a solution of compound **32** in DCM at -78°C.; slowly warming to 25°C and maintaining at 25°C for 12h; purifying crude compound **VII** by flash chromatography on silica gel; 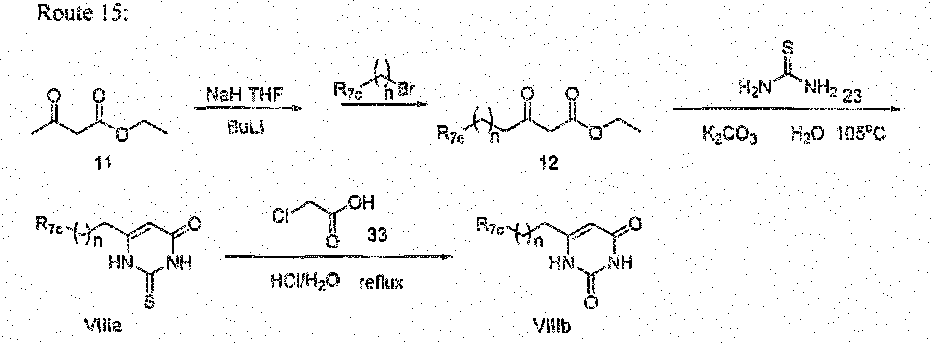
wherein, R_{7c} is independently methyl, or isopropyl,
subscript n is an integer selected from 6-12;
adding NaH to a solution of compound **11** in THF at 0°C, after 10 minutes, adding *n*-BuLi; after stirring for 10 minutes, adding the bromide; stirring the reaction mixture at 0°C for 14 h; purifying by flash chromatography on silica gel to afford compound **12;**
Suspending compound **23** in water and stirring at 70°C until said compound **23** was completely dissolved; adding K₂CO₃ and compound **12** and heating at 105°C in an open vessel; removing the heating after the solvent was completely evaporated, and cooling to room temperature; dissolving the solid in water to obtain a white slurry, adding HCl (1N) to the white slurry to adjust the pH to acidic, forming a white sticky solid, then removing the supernatant, washing with water (5 mL) 3 times, purifying by flash chromatography on silica gel to afford compound **VIIIa;**
adding compound 33 dissolved in a small amount of water slowly dropwise to a solution of compound **VIIIa** in THF/H₂O(2:1); heating to reflux for 6h, then, adding hydrochloric acid, maintaining refluxing for 12h, concentrating; purifying by flash chromatography on silica gel to afford compound **VIIIb;** 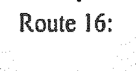 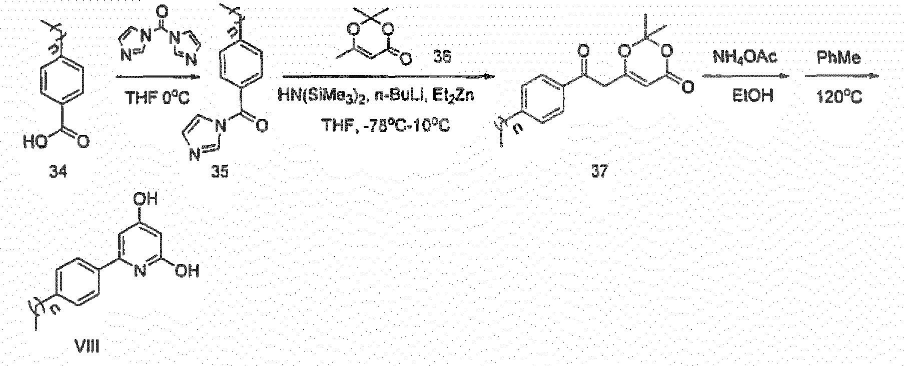
wherein, subscript n is an integer selected from 1-4;
reacting 1,1'-carbonyldiimidazole with compound **34** in THF to provide compound 35;
adding *n*-butyllithium dropwise to a solution of hexamethyldisilazane in THF at -78°C to prepare lithium hexamethyldisilazide, then adding diethylzinc, compound **35** and **36;** after the reaction, purifying by flash chromatography on silica gel to afford compound **37;**
reacting ammonium acetate with compound **37** in ethanol, maintaining at 25°C for 3h; concentrating, then adding toluene and heating to reflux for 3h; purifying crude compound **VIII** by flash chromatography on silica gel to afford a yellow solid; 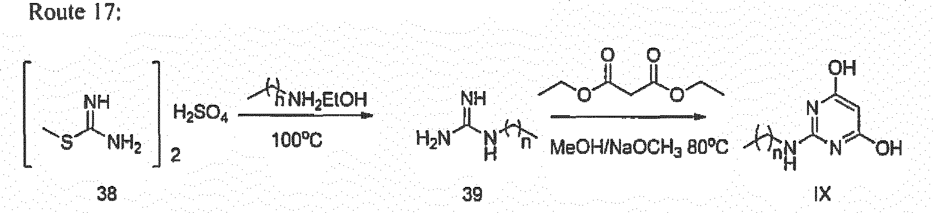
adding amino compound to a solution of compound **38** in ethanol, heating to reflux at 100 °C for 16h to obtain compound **39;**
adding compound **39** and diethyl malonate to a suspension of sodium in methanol, heating to reflux for 12 hours; concentrating the reaction mixture, followed by the addition of a small amount of water; adjusting the pH to acidity and forming a white precipitate, then filtering and purifying filter residue with flash chromatography on silica gel to afford compound **IX**;

### DETAILED DESCRIPTION

The following examples are provided to describe the invention in further detail. These examples are only used for illustration of the present invention without intended to limit the scope of the invention.

### Preparation Examples of the compounds

In the following Preparation Examples, NMR was measured using a Mercury-Vx 300M instrument manufactured by Varian, NMR calibration: δ H 7.26 ppm (CDCl₃), 2.50 ppm (DMSO-*d₆*), 3.15 ppm (CD₃OD); the reagents were mainly provided by Shanghai Chemical reagent Co., Ltd; the silica gel plate (Model No.: HSGF 254) used in TLC thin layer chromatography was produced by HuiyouSilica Gel Development Co., Ltd, Yantai, Shandong; silica gel used in the normal phase column chromatography for compound purification was produced by the branch of Ocean chemical Plant in Qingdao, Shandong, Model No.: zcx-11, 200-300 mesh.

### Preparation Example 1(Compound No.: LY214-5)

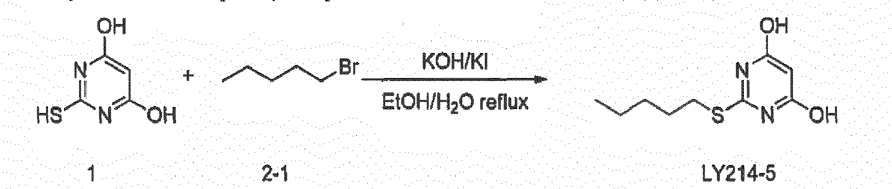

KI (11.3 mg, 0.069 mmol, 0.1 eq.) was added to a solution of compound 1 (100 mg, 0.69 mmol, 1.0 eq.) in EtOH/H₂O (10 mL/5 mL), and compound 2-1 (310 mg, 0.26 mL, 3.0 eq.) was slowly added. The reaction mixture was heated at 80°C for 6 hours. After the reaction was complete which was monitored by TLC, the residue was added dilute hydrochloric acid (1M, 10mL), and extracted with ethyl acetate for three times. The combined organic layers were washed with brine, dried, filtered, concentrated and purified using flash column chromatography (DCM : MeOH = 30 : 1) to give **LY214-5** (white solid, 12 mg, 8%). ¹H NMR (DMSO-*d₆*) δ12.1(s, 2H), 5.11(s,1H), 3.08(t, *J*=6.0Hz, 2H), 1.61(m, 2H), 1.36(m, 2H), 1.28(m, 2H), 0.87(t, *J*=6.3Hz, 3H).

The following compound was synthesized in the same manner:

| **Cpd#** | **Chemical structure** | **¹H NMR (300 MHz) data** |
|---|---|---|
| **LY214-5** | 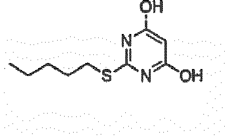 | (DMSO-d₆) δ12.1(s, 2H), 5.11(s,1H), 3.08(t, *J*=6.0Hz, 2H), 1.61(m, 2H), 1.36(m, 2H), 1.28(m, 2H), 0.87(t, *J*=6.3Hz, 3H) |
| **LY228-6a** | 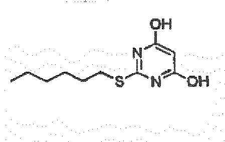 | (DMSO-d₆) δ12.1(s, 2H), 5.11(s,1H), 3.08(t, *J*=6.0Hz, 2H), 1.61(m, 2H), 1.36(m, 2H), 1.28(m, 4H), 0.87(t, *J*=6.3Hz, 3H) |
| **LY228-6b** | 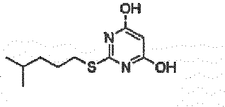 | (DMSO-d₆) δ12.1(s, 2H), 5.11(s,1H), 3.00(t, *J*=6.0Hz, 2H), 1.62(m, 2H), 1.32(m, 3H), 0.87(t, *J*=6.3Hz, 6H) |
| **LY242-7** | 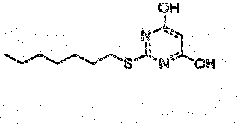 | (DMSO-d₆) δ12.1(s, 2H), 5.11(s,1H), 3.08(t, *J*=6.0Hz, 2H), 1.61(m, 2H), 1.36(m, 2H), 1.28(m, 6H), 0.87(t, *J*=6.3Hz, 3H) |
| **LY256-8** | 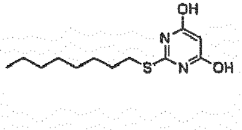 | (CD₃OD-d₄)δ12.1(s, 2H), 5.12(s,1H), 3.11(t, *J*=6.0Hz, 2H), 1.62(m, 2H), 1.41(m, 2H), 1.30(m, 8H), 0.87(t, *J*=6.3Hz, 3H) |
| **LY312-12** | 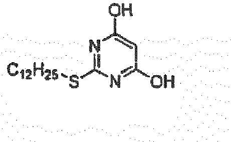 | (DMSO-d₆) δ12.1(s, 2H), 5.11(s,1H), 3.07(t, *J*=6.9Hz, 2H), 1.59(m, 2H), 1.26(m, 2H), 1.24(m, 18H), 0.87(t, *J*=6.3Hz, 3H) |
| **LY340-14** | 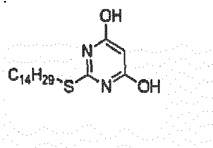 | (DMSO-d₆) δ12.1(s, 2H), 5.11(s,1H), 3.07(t, *J*=6.9Hz, 2H), 1.59(m, 2H), 1.26(m, 2H), 1.24(m, 20H), 0.87(t, *J*=6.3Hz, 3H) |
| **LY368-16** | 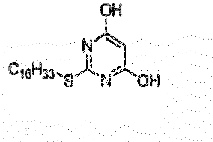 | (DMSO-d₆) δ12.1(s, 2H), 5.11(s,1H), 3.07(t, *J*=6.9Hz, 2H), 1.59(m, 2H), 1.26(m, 2H), 1.24(m, 24H), 0.87(t, *J*=6.3Hz, 3H) |
| **LY196** | 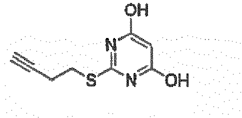 | (DMSO-d₆) δ 11.82 (s, 2H), 5.15 (s, 1H), 3.21 (t, *J* = 6.3 Hz, 2H), 2.92 (s, 1H), 2.55 (m, 2H). |
| **LY210** | 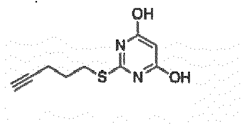 | (DMSO-d₆)δ11.68 (s, 2H), 5.06 (s, 1H), 3.16 - 3.06 (t, *J* = 6.9 Hz, 2H), 2.82 (s, 1H), 1.87 - 1.74 (m, 2H), 1.24 (m, 2H) |
| **LY226** | 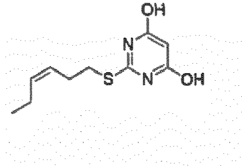 | (DMSO-d₆) δ 11.53 (s, 1H), 5.42 (d, *J* = 8.7 Hz, 1H), 5.39 (d, *J* = 7.2 Hz, 1H), 5.12 (s, 1H), 3.11 (t, *J* = 7.2 Hz, 2H), 2.38 (q, *J* = 6.9 Hz, 2H), 1.96-2.05 (m, 2H), 0.91 (t, *J* = 7.5 Hz, 3H). |
| **LY212** | 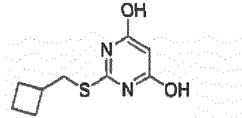 | (DMSO-d₆) δ 11.30 (s, 2H), 5.10 (s, 1 H), 3.19 (d, *J* = 7.8 Hz, 2H), 1.98-2.05(m, 1H), 1.90 - 1.61 (m, 6H). |
| **LY225-a** | 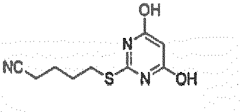 | (DMSO-d₆) δ12.5(s, 2H), 5.11(s,1H), 3.12(t, *J*=6.0Hz, 2H), 2.56(m, 2H), 1.60(m, 4H) |
| **LY272** | 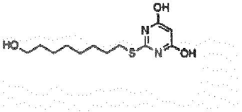 | (DMSO-d₆) δ 12.16 (s, 2H), 5.11 (s, 1H), 4.32 (s, 1H), 3.37 (t, *J* = 6.3 Hz, 2H), 3.08 (t, *J* = 7.2 Hz, 2H), 1.70 - 1.50 (m, 2H), 1.37 (s, 4H), 1.26 (s, 6H) |
| **LY248** | 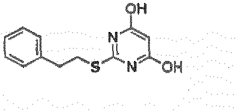 | (DMSO-d₆) δ 11.5 (s, 2H), 7.30 (s, 5H), 5.13 (s, 1H), 3.40-3.34(m,2H),3.00 - 2.87 (m, 2H). |
| **LY262-a** | 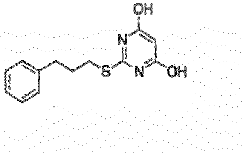 | (DMSO-d₆) δ 11.25 (s, 2H), 7.27 (m, 2H), 7.21 (m, 3H), 5.13 (s, 1H), 3.10 (t, *J* = 6.9Hz, 2H), 2.75 - 2.60 (m, 2H), 2.01 - 1.83 (m, 2H). |
| **LY276** | 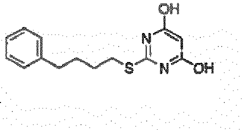 | (DMSO-d₆) δ 11.26 (s, 2H), 7.25 (m, 2H), 7.20 (m, 3H), 5.10 (s, 1 H), 3.08 (t, *J* = 7.2 Hz, 2H), 2.61 -2.53 (q, *J* = 6.6 Hz, 2H), 1.71 - 1.53 (m, 4H), 1.35-1.43 (m, 2H). |
| **LY290-a** | 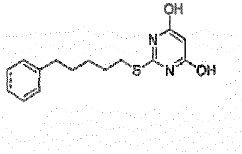 | (DMSO-d₆) δ 12.47(s, 1H), 7.31 - 7.22 (m, 2H), 7.19 (m, 3H), 5.93 (s, 1H), 3.13 (t, *J* = 5.7, 2H), 2.61 (q, *J* = 6.3 Hz, 2H), 2.14 (m, 4H), 1.66 (s, 3H). |
| **LY266-a** | 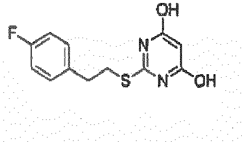 | (DMSO-d₆) δ11.41(s, 2H), 7.32 (d, *J=* 6.4 Hz, 2H), 7.13 (t, *J* = 8.4 Hz, 2H), 5.28 (s, 1H), 3.01 - 2.88 (m, 2H), 2.99 - 2.88 (m, 2H). |
| **LY266-b** | 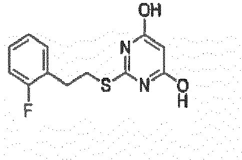 | (DMSO-d₆)δ11.33 (s, 2H), 7.38 (d, *J* = 7.5 Hz, 1H), 7.27 (s, 1H), 7.16 (t, *J* = 8.7 Hz, 2H), 5.15 (s, 1H), 3.40 - 3.33 (m, 2H), 3.00 (t, *J* = 7.2 Hz, 2H). |
| **LY266-c** | 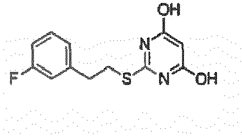 | (DMSO-d₆)δ 11.87 (s, 2H), 7.35 (d, *J* = 6.9Hz, 1H), 7.15 (t, *J* = 8.1 Hz, 2H), 7.04 (s, 1H), 3.38 - 3.32 (m, 2H), 2.97 (t, *J* = 7.7 Hz, 2H). |
| **LY278** | 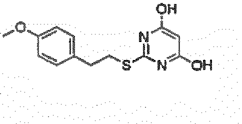 | (DMSO-d₆) δ11.53(s, 2H), 7.21 (d, *J* = 6.4 Hz, 2H), 6.82 (d, *J* = 6.4 Hz, 2H), 5.28 (s, 1H), 3.78 (s, 3H), 3.01 - 2.88 (m, 2H), 2.82 - 2.88 (m, 2H). |
| **LY312** | 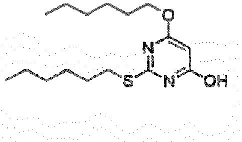 | (DMSO-d₆) δ 12.2(s, 1H), 5.36 (s, 1H), 4.18 (t, *J* = 6.6 Hz, 2H), 3.07 (t, *J* = 7.2 Hz, 2H), 1.63 (dd, *J* = 12.9, 6.3 Hz, 4H), 1.43 - 1.32 (m, 4H), 1.29 (d, *J* = 3.3 Hz, 8H), 0.87 (t, *J* = 4.8 Hz, 6H). |

### Preparation Example 2 (compound No.: LY224-a)

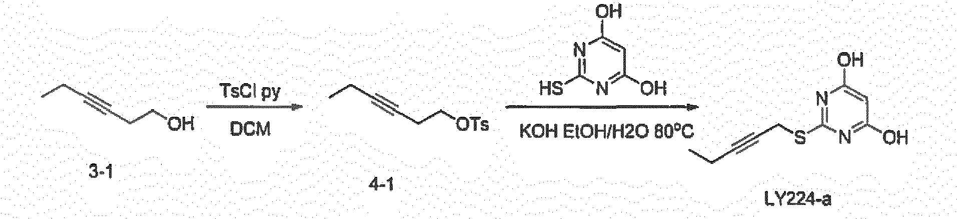

Compound **3-1** (0.1 mL, 1.02 mmol, 1 eq.) was dissolved in dry DCM (5 mL), and pyridine was added (104 mg, 1.3 mmol, 1.3 eq.). The reaction mixture was cooled to 0°C, then a solution of TsCl (214 mg, 1.12 mmol, 1.1 eq.) in anhydrous DCM (5 mL) was slowly added dropwise. The reaction mixture was allowed to warm to 20 °C and stirred for 12 h monitored by TLC. Upon completion, the residue was concentrated and purified by a flash chromatography on silica gel (DCM:MeOH=30:1) to give compound **4-1** (colorless oil, 145mg, 56%). ¹H NMR (300 MHz, CDCl₃) δ 7.80 (d, *J* = 5.1 Hz, 2H), 7.34 (d, *J* = 8.1 Hz, 2H), 4.05 (t, *J* = 7.2 Hz, 2H), 2.50 (m, 2H), 2.45 (s, 3H), 2.08 (m, 2H), 1.06 (t, *J* = 7.5 Hz, 3H).

Compound **1** (67 mg, 0.47 mmol, 1.0 eq.) was dissolved in EtOH/H₂O (10 mL/5 mL) and compound **4-1** (145 mg, 0.52 mmol, 1.1 eq.) was added slowly. The reaction was carried out at 80°C for 6 h monitored by TLC. Upon completion, the mixture was added diluted hydrochloric acid (1M, 10 mL) followed by extracted with ethyl acetate (15 mL) for three times. The combined organic extracts were washed with brine, dried, filtered, concentrated and chromatographed (DCM:MeOH=30:1) to give **LY224-a** (white solid, 44 mg, 42%). ¹H NMR (300 MHz, DMSO-*d*₆) δ 11.36 (s, 1H), 5.12 (s, 1H), 3.18 (t, *J* = 6.3 Hz, 2H), 2.52-2.55 (m, 2H), 2.21 - 2.03 (m, 2H), 1.03 (t, *J* = 7.5 Hz, 3H).

### Preparation Example 3 (compound No.: LY224-b)

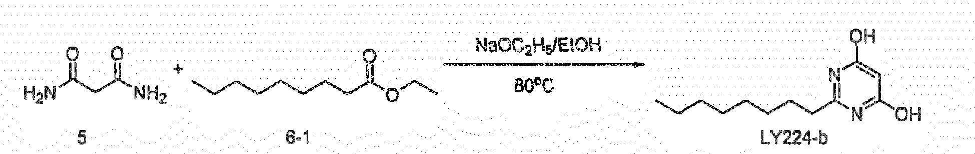

Sodium (250mg) was dissolved in ethanol (8mL), then added compound 5 (500mg, 4.9mmol, 1eq). After the solid was dissolved, compound **6-1** (1.1 g, 5.89 mmol, 1.2 eq.) in ethanol (5 mL) was slowly added dropwise. A white solid was precipitated. After heated to reflux for 3 hours, the reaction mixture was then cooled and filtered. The filtrate was concentrated and added 3 mL of H₂O to dissolve the solid. A dilute hydrochloric acid (2M) was slowly added dropwise to adjust pH to 3-5. A white precipitation was filtered. The filter cake was added 5mL of methanol, stirred for 1h, filtered and dried to obtain compound **LY224-b** (white solid, 1.0g, 91%). ¹H NMR (300 MHz, DMSO-*d₆*) δ 11.64 (s, 2H), 5.02 (s, 1H), 2.52(t, *J* = 7.5 Hz, 2H), 1.62 (q, *J* = 6.6 Hz 2H), 1.25 (m, 10H), 0.84 (t, *J* = 6.6 Hz, 3H).

The following compound was synthesized in the same manner:

| **Cpd#** | **Chemical structure** | **¹H NMR (300 MHz) data** |
|---|---|---|
| **LY182** | 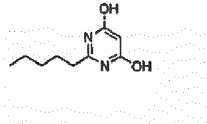 | (CD₃OD-d₄) δ11.64 (s, 2H), 5.25 (s, 1H), 2.59 ((t, *J* = 8.1 Hz ,2H), 174 (q, *J* = 7.5 Hz 2H), 1.42 - 1.31 (m, 4H), 0.93 (t, *J* = 6.6 Hz, 3H). |
| **LY196-b** | 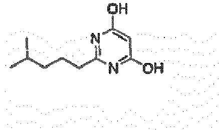 | (CD₃OD-d₄) δ11.64 (s, 2H), 5.25 (s, 1H), 2.56 (t, *J* = 7.8 Hz ,2H), 1.74 (q, *J* = 7.5 Hz 2H), 1.57 (m, 1H), 1.25 (m,2H), 0.91 (d, *J* = 3.3 Hz, 3H), 0.89 (d, *J* = 3.3 Hz ,3H). |
| **LY196** | 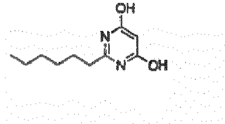 | (CD₃OD-d₄) δ11.64 (s, 2H), 5.25 (s, 1H), 2.59(t, *J* = 7.5 Hz, 2H), 1.73 (q, *J* = 7.5 Hz 2H), 1.34 (m, 6H), 0.91 (t, *J* = 6.9 Hz, 3H). |
| **LY210-b** | 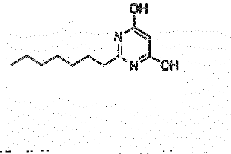 | (CD₃OD-d₄) δ11.64 (s, 2H), 5.25 (s, 1H), 2.59(t, *J* = 7.5 Hz, 2H), 1.73 (q, *J* = 7.5 Hz 2H), 1.34 (m, 8H), 0.91 (t, *J* = 6.9 Hz, 3H). |
| **LY224-b** | 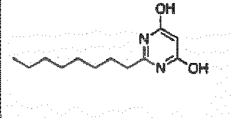 | (CD₃OD-d₄) δ 11.64 (s, 2H), 5.02 (s, 1H), 2.52(t, *J* = 7.5 Hz, 2H), 1.62 (q, *J* = 6.6 Hz 2H), 1.25 (m, 10H), 0.84 (t, *J* = 6.6 Hz, 3H). |
| **LY238** | 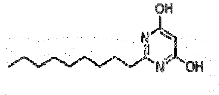 | (CD₃OD-d₄) δ11.64 (s, 2H), 5.25 (s, 1H), 2.54 (t, *J* = 7.5 Hz 2H), 1.73 (q, *J* = 6.6 Hz,2H), 1.30 (m, 12H), 0.89 (t, *J* = 6.9 Hz, 3H). |

### Preparation Example 4 (compound No.: LY244)

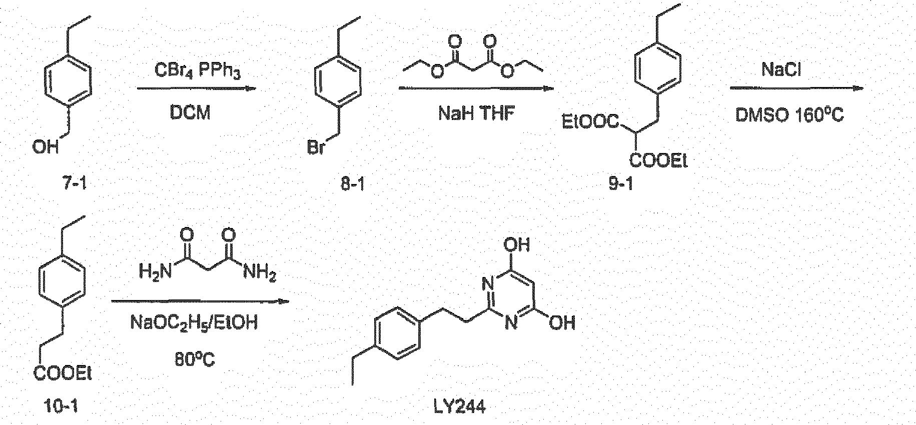

Compound **7-1** (0.5 mL, 3.68 mmol, 1 eq.) was dissolved in dry DCM (20 mL), then added triphenylphosphine (1.2 g, 4.41 mmol, 1.2 eq.), carbon tetrabromide (1.4 g, 4.41 mmol, 1.2 eq.) and stirred at 20°C for 12 h. Upon the reaction was completion monitored by TLC, the solvent was removed under reduced pressure, and the residue was chromatographed (petroleum ether) to afford compound **8-1** (colorless oil, 310 mg, 59%). ¹H NMR (300 MHz, CDCl₃) δ 7.80 (d, *J* = 8.1 Hz, 2H), 7.34 (d, *J* = 8.1 Hz, 2H), 4.05 (s, 2H), 2.50 (m, 2H), 2.45 (q, *J* = 8.1 Hz, 3H), 2.08 (m, 2H), 1.06 (t, *J* = 7.5 Hz, 3H).

Diethyl malonate (91 mg, 0.63 mmol, 1 eq.) was dissolved in THF (10 mL), cooled to -78°C. NaH (19.7 mg, 0.82 mmol, 1.1 eq.) was added to the solution and stirred for 10 minutes. Then, compound **8-1** (100 mg, 0.69 mmol, 1.1 eq.) was slowly dripped in. The reaction was gradually warmed to 20 °C. and stirred for 12 h monitored by TLC. After reaction completion, the reaction was quenched with water (5 mL), extracted with ethyl acetate (10 mL) for three times, and washed twice with water (5 mL). The combined organic layers were washed with brine, dried over anhydrous sodium sulfate, filtered and purified by a flash chromatography on silica gel to obtain compound **9-1** (colorless oil, 52 mg, 41%). ¹H NMR (300 MHz, CDCl₃) δ 7.10 (m, 4H), 4.25 - 4.10 (m, 4H), 3.67 (t, *J* = 6.9 Hz, 1H), 3.17 (d, *J* = 7.8 Hz, 2H), 2.60 (q, *J* = 13.8 Hz, 2H), 1.27 (t, *J* = 7.2 Hz, 3H), 1.20 (t, *J* = 7.2, 6H).

A solution of compound **9-1** (130 mg, 0.63 mmol, 1 eq.) and NaCl (74 mg, 1.26 mmol, 2 eq.) in DMSO (2 mL) was heated at 160 °C for 3 h. The reaction mixture was added water (5 mL) and extracted three times with ethyl acetate (5 mL). The organic phase was washed three times with water (5 mL). Then, the organic phase was combined and washed with brine, dried over anhydrous sodium sulfate, and purified by a flash chromatography on silica gel to obtain compound 10-1 (colorless oil, 56 mg, 43%). ¹H NMR (300 MHz, CDCl₃) δ 7.12 (m, 4H), 4.14 (q, *J* = 14.4 Hz, 2H), 2.91(t, *J* = 8.4 Hz, 2H), 2.59 (t, *J* = 8.4 Hz, 2H), 2.54 (q, *J* = 13.8 Hz, 2H), 1.28((t, *J* = 7.2 Hz, 3H), 1.22 ((t, *J* = 7.2 Hz, 3H).

Sodium (250 mg) was dissolved in ethanol (8 mL), then added compound **2** (212 mg, 2.08 mmol, 1.1 eq.). After the solid was dissolved, compound **10-1** (390 mg, 1.89 mmol, 1 eq.) in ethanol (5 mL) was slowly added dropwise. A white solid was precipitated. After heated to reflux for 3h, the reaction was cooled to room temperature, filtered, concentrated, dissolved with 3mL of H₂O. A dilute hydrochloric acid (2M) was slowly added dropwise to the residue to adjust pH to 3-5. A white precipitation was filtered. The filter cake was added in 5mL of methanol, stirred for 1h, filtered and dried to obtain compound **LY244** (white solid, 147mg, 35%). ¹H NMR (300 MHz, DMSO) δ 11.50(s, 2H), 7.12 (m, 4H), 5.06 (s, 1H), 2.95 - 2.88 (t, *J* = 7.5 Hz ,2H), 2.78 - 2.69 (t, *J* = 8.1 Hz ,2H), 2.60 - 2.53 (q, *J* = 7.5 Hz ,2H), 1.15 (t, *J* = 7.5 Hz, 1H).

### Preparation Example 5 (compound No.: LY237 & LY238-d)

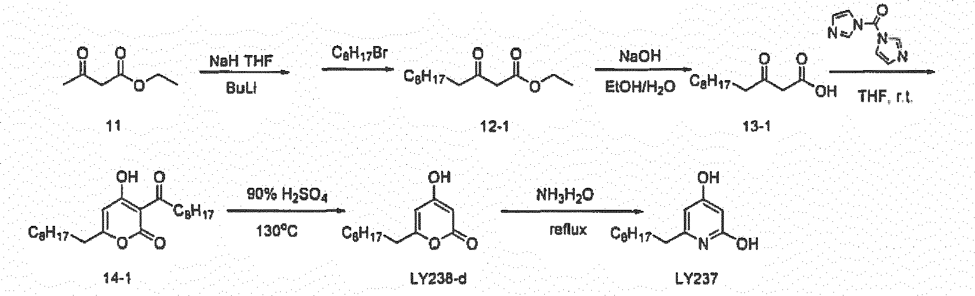

Compound **11** (1 mL, 7.7 mmol, 1 eq.) was added in dry THF (20 mL), cooled to 0°C, then added NaH (203 mg, 8.5 mmol, 1.1 eq.), stirred for 5 minutes, added *n*-butyllithium (5.3 mL, 8.5 mmol, 1.6M, 1.1 eq.). Stirred for another 5 minutes, bromide (1.3 mL, 7.7 mmol, 1 eq.) was added and stirred at 0°C for 12 h monitored by TLC. The reaction mixture turned into a yellow milky liquid. After full completion, the reaction was quenched with water (10 mL) and extracted three times with ethyl acetate (10 mL). The combined organic extracts were washed with brine, dried over anhydrous sodium sulfate, and purified by a flash chromatography on silica gel to give compound **12-1** (yellow oil, 647 mg, 34%). ¹H NMR (300 MHz, CDCl₃) δ 4.19 (q, *J* = 15 Hz, 2H), 3.42 (s, 2H), 2.53 (t, *J* = 7.2 Hz, 2H), 1.28 (m, 14H), 0.87 (t, *J* = 6.3 Hz, 3H). ¹H NMR (300 MHz, DMSO) δ 12.53 (s, 1H), 3.43 (s, 2H), 2.39 (t, *J* = 7.2 Hz, 2H), 1.23 (m, 14H), 0.85 (t, *J* = 6.3 Hz, 3H).

Compound **12-1** (647 mg, 2.67 mmol, 1 eq.) was dissolved in EtOH/H₂O (10/10 mL), then added NaOH (139 mg, 3.47 mmol, 1.3 eq.). The reaction was stirred at 20 °C for 12 h. The reaction mixture was washed twice with ethyl acetate (10 mL). The aqueous layer was adjusted to acidic (pH 3-5) with IN HCl. A white solid was precipitated, filtered and dried in vacuo to obtain compound **13-1** (white solid, 410 mg, 72%). ¹H NMR (300 MHz, DMSO) δ 12.53 (s, 1H), 3.43 (s, 2H), 2.39 (t, *J* = 7.2 Hz, 2H), 1.23 (m, 14H), 0.85 (t, *J* = 6.3 Hz, 3H).

Compound **13-1** (100 mg, 0.47 mmol, 1 eq.) was dissolved in anhydrous THF (10 mL), then added 1,1'-carbonyldiimidazole (106 mg, 0.65 mmol, 1.4 eq.) and stirred at 20 °C for 12 h. The reaction mixture was added H₂O (10 mL) and extracted three times with ethyl acetate (10 mL). The combined the organic layers were concentrated and added methanol (3 mL). The reaction was left to stand until white needle crystals were precipitated, then filtered to obtain compound **14-1** (white needle crystals, 28 mg, 16%). ¹H NMR (300 MHz, CDCl₃) δ12.34(s,1H), 5.91 (s, 1H), 3.07 (t, *J* = 7.2 Hz, 2H), 2.47 t, *J* = 7.8 Hz, 2H), 1.65 (m, 4H), 1.26 (m, 24H), 0.88 (t, *J* = 6.7, 5.7 Hz, 6H).

Compound 14-1 (14mg, 0.037mmol, 1eq.) was dissolved in 90% H₂SO₄ (5mL), heated at 130°C for 1h. The reaction mixture was added ethyl acetate (5mL). The phases were separated, and the organic layer was concentrated in vacuo. The crude product was purified by a flash chromatography on silica gel to give **LY238-d** (white solid, 6mg, 67 %). ¹H NMR (300 MHz, CDCl₃) δ 5.96 (s, 1H), 5.57 (s, 1H), 5.30 (s, 1H), 2.46 (t, *J* = 7.5 Hz, 2H), 1.62 (m, 2H), 1.26 (m, 12H), 0.87 (d, *J* = 6.3Hz, 3H).

A mixture of compound **LY238-d** (26mg, 0.1 mmol, 1eq.) in 30% ammonium hydroxide (5mL) was heated to reflux at 100 °C for 14h. A dilute hydrochloric acid (1M) was added to adjust the pH to 3-4. A white solid was precipitated, filtered, and dried to obtain compound **LY237** (white solid, 20 mg, 83%). ¹H NMR (300 MHz, DMSO) δ10.85(s,1H),10.27(s,1H), 5.58 (s, 1H), 5.33 (s, 1H), 2.33 (t, *J* = 7.5 Hz, 2H), 1.51 (q, *J* = 6.6 Hz,2H), 1.24 (m, 12H), 0.85 (t, *J* = 6.0 Hz, 3H).

The following compound was synthesized in the same manner:

| **Cpd#** | **Chemical structure** | **¹H NMR (300 MHz) data** |
|---|---|---|
| **LY223** | 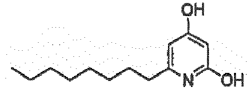 | ¹H NMR (300 MHz, DMSO) δ 1 0.85(s, 1H), 10.27(s, 1 H), 5.58 (s, 1H), 5.33 (s, 1H), 2.33 (t, *J* = 7.5 Hz, 2H), 1.51 (q, *J* = 6.6 Hz,2H), 1.24 (m, 10H), 0.85 (t, *J* = 6.0 Hz, 3H). |
| **LY209** | 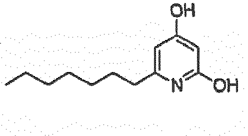 | ¹H NMR (300 MHz, DMSO) δ 10.85 (s, 1H), 10.25 (s, 1H), 5.58 (s, 1 H), 5.32 (s, 1H), 2.33 (t, *J* = 7.5 Hz, 2H), 1.51 (m, 2H), 1.24 (m, 10H), 0.86 (t, *J* = 6.9 Hz, 3H). |

### Preparation Example 6(compound No.: LY250)

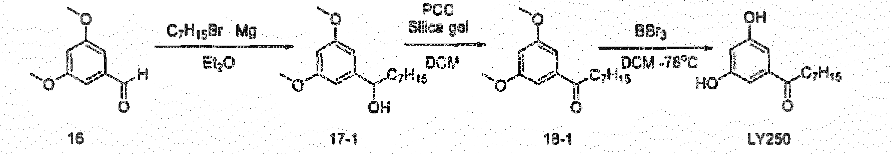

1-bromoheptane (231 mg, 1.2 mmol, 1 eq.) was slowly added dropwise under N₂ atmosphere to a suspension of magnesium (58 mg, 2.4 mmol, 2 eq.) in anhydrous ether (5 mL). The reaction was refluxed at 50°C for 45 minutes, and slowly added compound **16** (200 mg, 1.2 mmol, 1 eq.) in anhydrous ether (10 mL), then, refluxed at 50°C for 3 h. After completion of the reaction monitored by TLC, the reaction was slowly quenched with water (5 mL) and extracted three times with ethyl acetate (10 mL). The combined organic layers were washed with brine, dried, concentrated, and chromatographed (petroleum ether: ethyl acetate=10:1) to give compound **17-1** (colorless oil, 200 mg, 60%). ¹H NMR (300 MHz, CDCl₃) δ 6.51 (d, *J* = 2.1Hz, 2H), 6.37 (t, *J* = 2.4Hz, 1H), 4.64 - 4.54 (m, 1H), 3.78 (s, 6H), 1.57 (m, 2H), 1.26 (m, 12H), 0.85 (t, *J* = 6.6 Hz, 3H).

Compound **17-1** (220 mg, 0.79 mmol, 1 eq.) was dissolved in dry DCM (10 mL), then added PCC (507 mg, 2.36 mmol, 3 eq.) and silica gel (880 mg), and stirred at 20 °C for 14 h monitored by TLC. After full completion, the reaction mixture was filtered, concentrated, and chromatographed (petroleum ether : ethyl acetate=10:1) to obtain compound **18-1** (white solid, 161 mg, 73%). ¹H NMR (300 MHz, CDCl₃) δ 7.09 (d, *J* = 2.1 Hz, 2H), 6.63 (t, *J* = 2.1 Hz, 1H), 3.83 (s, 6H), 2.87 (t, *J* = 7.5 Hz, 2H), 1.77 - 1.62 (m, 2H), 1.30 (m, 12H), 0.88 (t, *J* = 6.6 Hz, 3H).

Compound **18-1** (157 mg, 0.56 mmol, 1 eq.) was dissolved in dry DCM (5 mL) and cooled to -78°C, then slowly added BBr₃ (2M 0.85 mL, 1.68 mmol, 3 eq.) dropwise. The reaction was slowly warmed to 20°C and stirred for 14h monitored by TLC. The reaction was quenched by the dropwise addition of water (5 mL), and extracted three times with ethyl acetate (10 mL). The combined organic extracts were washed with brine, dried, concentrated. The residue was purified by a flash chromatography on silica gel (petroleum ether : ethyl acetate=3: 1) to afford compound **LY250** (colorless oil, 100 mg, 71%). ¹H NMR (300 MHz, MeOD-*d₄*) δ 6.87 (d, *J* = 2.1 Hz, 2H), 6.47 (m, 1H), 2.90 (t, *J* = 7.2 Hz, 2H), 1.66 (m, 2H), 1.32 (m, 10H), 0.89 (t, *J* = 6.9 Hz, 3H).

### Preparation Example 7 (compound No.: LY234)

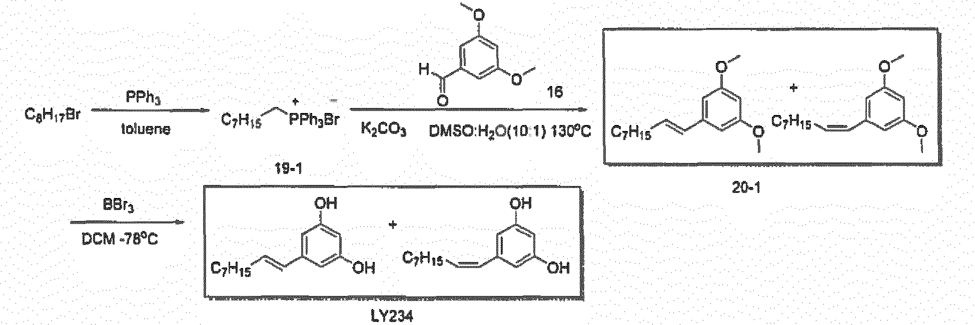

1-bromooctane (1 mL, 5 mmol, 1 eq.) was dissolved in toluene (20 mL), then added triphenylphosphine (1.6 g, 6 mmol, 1.2 eq.) and refluxed at 120°C for 12 h. The reaction mixture was concentrated and diluted with *n*-hexane (20 mL). A white sticky solid was precipitated. The two phase was separated and the crude product **19-1** was triturated three times with petroleum ether /ethyl acetate (20 mL/10 mL) and dried in vacuo, which was used without further purification.

Compound **19-1** (460 mg, 1.01 mmol, 1.2 eq.) was dissolved in DMSO/H₂O (5 mL/0.5 mL), then added compound 18 (139 mg, 0.84 mmol, 1 eq.), potassium carbonate (232 mg, 1.68 mmol, 2 eq.), refluxed at 130°C for 12 h monitored by TLC. After then, the reaction was extracted three times with ethyl acetate (5 mL), washed three times with H₂O (5 mL). The combined organic layers were washed with brine, dried, and concentrated. The residue was purified by a flash chromatography on silica gel (petroleum ether : ethyl acetate=50 : 1) to give compound **20-1** (colorless oil, 230 mg, 89%). (E/Z=1.2) E ¹H NMR (300 MHz, CDCl₃) δ 6.50 (d, *J* = 2.1 Hz, 2H), 6.34 (t, *J* = 2.4 Hz, 1H), 6.31 (d, *J* = 15.6 Hz, 1H), 6.28 (m, 1H), 3.79 (s, 3H), 2.19 (q, *J* = 13.2 Hz, 2H), 1.44 (m, 2H), 1.28 (m, 8H), 0.88 (t, *J* = 6.6 Hz, 4H). Z ¹H NMR (300 MHz, CDCl₃) δ6.43 (d, *J* = 2.1 Hz, 1H), 6.23 (t, *J* = 6.4 Hz, 1H), 6.34 (d, *J* = 11.4 Hz, 1H), 5.67 (m, 1H), 3.79 (s, 3H), 2.33 (q, *J* = 14.1 Hz, 2H), 1.44 (m, 2H), 1.44 (m, 2H), 1.28 (m, 8H), 0.88 (t, *J* = 6.6 Hz, 4H).

Compound **20-1** (476 mg, 1.8 mmol, 1 eq.) was dissolved in dry DCM (10 mL) and cooled to -78°C, then slowly added dropwise BBr₃ (2M 2.7 mL, 5.4 mmol, 3 eq.). The reaction was slowly warmed to 20°C and stirred for 14h monitored by TLC. The reaction was quenched by the dropwise addition of water (10 mL), and extracted three times with ethyl acetate (15 mL). The combined organic extracts were washed with brine, dried, concentrated. The residue was purified by a flash chromatography on silica gel (petroleum ether: ethyl acetate = 3 : 1) to obtain compound **LY234** (a yellow oil, 320 mg, 76%). ¹H NMR (300 MHz, CDCl₃) E δ 6.48 - 6.30 (m, 3H), 6.30 - 6.09 (m, 2H), 2.16 (m, 2H), 1.42 (m, 10H), 0.93 (t, *J* = 7.5 Hz, 3H). Z δ 6.48 - 6.30 (m, 3H), 6.35 (m, 1H), 5.63(m, 1H), 2.29 (m, 2H), 1.42 (m, 10H), 0.93 (t, *J* = 7.5 Hz, 3H).

### Preparation Example 8 (compound No.: LY236)

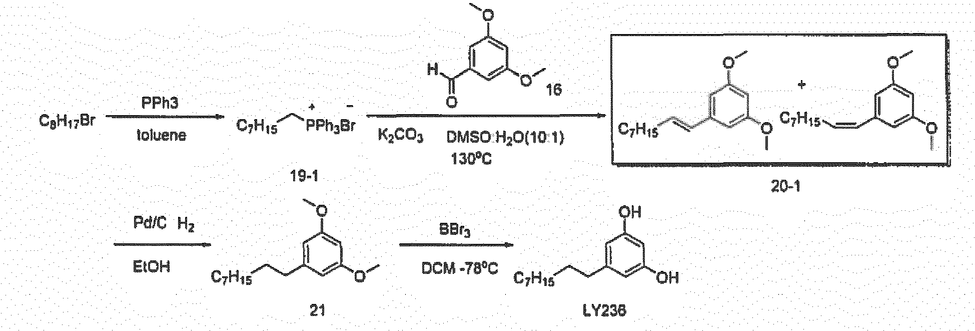

Compound **19-1, 20-1** were prepared in a manner analogous to Example 7;
A mixture of compound **20-1** (100 mg, 0.38 mmol), EtOH (10 mL), Pd/C (10 mg) was stirred under an atmosphere of H₂ at 20° C for 14h, monitored by TLC. After the reaction completion, the mixture was purged with nitrogen, and filtered. The filtrate was concentrated in vacuo and purified by a flash chromatography on silica gel (petroleum ether: ethyl acetate = 50:1) to afford compound 21 (colorless oil, 54 mg, 54%). ¹H NMR (300 MHz, CDCl₃) δ 6.35 (d, *J* = 2.1 Hz, 2H), 6.31 - 6.28 (t, *J* = 2.1 Hz, 1H), 3.78 (s, 6H), 2.54 (t, *J* = 7.5 Hz, 2H), 1.58 (m, 4H), 1.28 (m, 6H), 0.88 (t, *J* = 6.6 Hz, 3H).

Compound 21 (264 mg, 0.19 mmol, 1 eq.) was dissolved in dry DCM (5 mL), cooled to -78°C, then slowly added dropwise BBr₃ (2M 0.27 mL, 0.54 mmol, 3 eq.). The reaction mixture was slowly warmed to 20°C and maintained at 20°C for 14h monitored by TLC. The reaction was quenched by the dropwise addition of water (5 mL), and extracted three times with ethyl acetate (10 mL). The combined organic extracts were washed with brine, dried, concentrated. The residue was purified by a flash chromatography on silica gel (petroleum ether: ethyl acetate = 3 : 1) to obtain compound **LY236** (yellow solid, 16 mg, 70%). ¹H NMR (300 MHz, CDCl₃) δ 6.24 (d, *J* = 2.1 Hz, 2H), 6.22 - 6.13 (m, 1H), 4.73 (s, 2H), 2.48(t, *J* = 7.8 Hz, 2H), 1.56 (m, 2H), 1.26 (m, 12H), 0.88 (t, *J* = 6.6 Hz, 3H).

### Preparation Example 9 (compound No.: LY290-b)

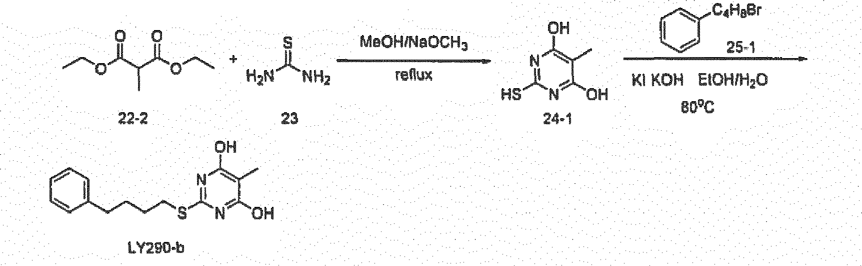

Na (1.2 g) was slowly dissolved in 17 mL of methanol, thereafter, added compound **23** (3.8 g, 0.05 mol, 1 eq.) in methanol (17 mL) and compound **22-2** (8.7 g, 0.05 mol, I eq.). A white solid was precipitated. The reaction mixture was heated to reflux for 16h, then, cooled to 50°C, adjusted the pH to acidity with hydrochloric acid (1M, 25mL). The white solid was gradually dissolved. The mixture was filtered to remove impurities, cooled to 0°C, and was left to stand for 12h. The crystal was formed, filtered, washed with ice water and dried to give compound **24-1** (2.08 g, white solid, 26.3%).

Compound **24-1** (100 mg, 0.60 mmol, 1.0 eq.) was dissolved in EtOH/H₂O (10 mL/5 mL), added KI (11.3 mg, 0.069 mmol, 0.1 eq.), and then, slowly added compound 25-1 (381 mg, 1.80 mmol, 3.0eq.). The reaction was heated to 80°C for 6h, monitored by TLC. After the reaction was completed, the mixture was added dilute hydrochloric acid (1M, 10mL), followed by extracted three times with ethyl acetate (15mL). The combined organic extracts were washed with brine, dried, filtered, and concentrated. The residue was purified by a flash chromatography on silica gel (DCM: MeOH = 30: 1) to give compound **LY290-b** (white solid, 17 mg, 9.8%). ¹H NMR (300 MHz, DMSO) δ 11.31 (s, 2H), 7.26 (m, *J* = 7.2, 2H), 7.21 - 7.11 (m, 3H), 3.13 (t, *J* = 6.6, 2H), 2.59 (m, 2H), 1.71 (s, 3H), 1.69 - 1.60 (m, 4H).

### Preparation Example 10 (compound No.: LY274-a)

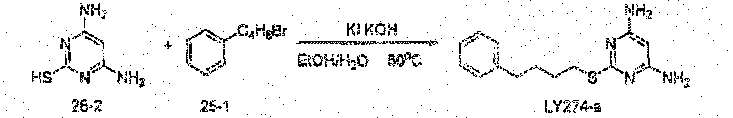

Compound **26-2** (100 mg, 0.70 mmol, 1.0 eq.) was dissolved in EtOH/H₂O (10 mL/5 mL), added KI (11.6 mg, 0.07 mmol, 0.1 eq.), and then, slowly added compound **25-1** (445 mg, 2.1 mmol, 3.0 eq.). The reaction was heated at 80 °C for 6h, monitored by TLC. After the reaction was completed, the mixture was added dilute hydrochloric acid (1M, 10mL), followed by extracted three times with ethyl acetate (15mL). The combined organic extracts were washed with brine, dried, filtered, and concentrated. The residue was purified by a flash chromatography on silica gel (DCM : MeOH = 30:1) to give compound **LY274-a** (white solid, 10 mg, 5.2%). ¹H NMR (300 MHz, DMSO) δ 7.26 (m, 2H), 7.19 (m, 3H), 6.02 (s, 4H), 5.12 (s, 1H), 3.00 (t, *J* = 6.6 Hz, 2H), 2.59 (q, *J* = 6.6 Hz, 2H), 1.62 (m, 4H).

The following compound was synthesized in the same manner:

| **Cpd#** | **Chemical structure** | **¹H NMR (300 MHz) data** |
|---|---|---|
| **LY274-a** | 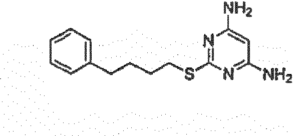 | ¹H NMR (300 MHz, DMSO) δ 7.26 (m, 2H), 7.19 (m, 3H), 6.02 (s, 4H), 5.12 (s, 1H), 3.00 (t, *J* = 6.6 Hz, 2H), 2.59 (q, *J* = 6.6 Hz, 2H), 1.62 (m, 4H). |
| **LY274-b** | 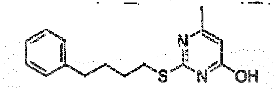 | ¹H NMR (300 MHz, DMSO) δ 12.47(s, 1H), 7.31 - 7.22 (m, 2H), 7.19 (m, 3H), 5.93 (s, 1H), 3.13 (t, *J* = 5.7, 2H), 2.61 (q, *J* = 6.3 Hz, 2H), 2.14 (m, 4H), 1.66 (s, 3H). |
| **LY275** | 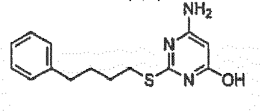 | ¹H NMR (300 MHz, DMSO) δ 11.434 (S, 1H), 7.30 - 7.23 (m, 2H), 7.19 (m, 3H), 6.40 (s, 2H), 4.87(s, 1H), 3.09 (t, *J* = 6.6 Hz, 2H), 2.59 (q, *J* = 6.6 Hz, 2H), 1.64 (m, 4H). |

### Preparation Example 11 (compound No.: LY328)

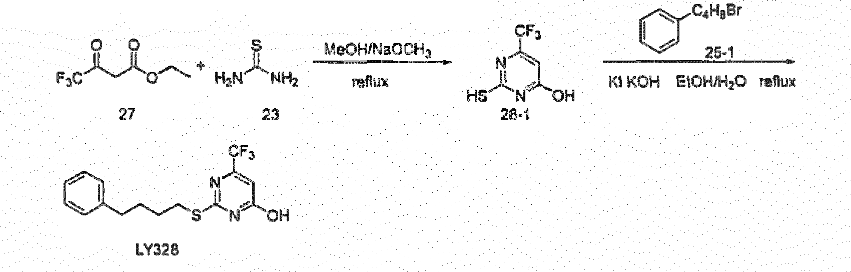

Na (600 mg) was slowly dissolved in 5 mL of methanol, thereafter, added compound **23** (760 mg, 0.01 mol, 1 eq.) in methanol (5 mL) and compound **27** (2.00 g, 0.01 mol, 1 eq.). The reaction mixture was heated to reflux for 16h, then, cooled to 50°C, adjusted the pH to acidity with hydrochloric acid (1M, 25mL). The white solid was gradually dissolved. The mixture was filtered to remove impurities, cooled to 0°C, and was left to stand for 12h. The crystal was formed, filtered, washed with ice water and dried to give compound **26-1** (1.00 g, white solid, 51.0%).

Compound **26-1** (100 mg, 0.50 mmol, 1.0 eq.) was dissolved in EtOH/H₂O (10 mL/5 mL), added KI (11.3 mg, 0.069 mmol, 0.1 eq.), and then, slowly added compound **25-1** (318 mg, 1.50 mmol, 3.0eq.). The reaction was heated at 80 °C for 6h, monitored by TLC. After the reaction was completed, the mixture was added dilute hydrochloric acid (1M, 10mL), followed by extracted three times with ethyl acetate (15mL). The combined organic extracts were washed with brine, dried, filtered, and concentrated. The residue was purified by a flash chromatography on silica gel (DCM : MeOH = 30:1) to give compound **LY328** (white solid, 80 mg, 67.2%). ¹H NMR (300 MHz, DMSO) δ 13.50 (s, 1H), 7.32 - 7.22 (m, 2H), 7.17 (m, 3H), 6.58 (s, 1H), 3.16 (t, *J* = 9.6 Hz, 2H), 2.60 (q, *J* = 6.9 Hz, 2H), 1.67 (m, 4H)

### Preparation Example 12 (compound No.: LY242)

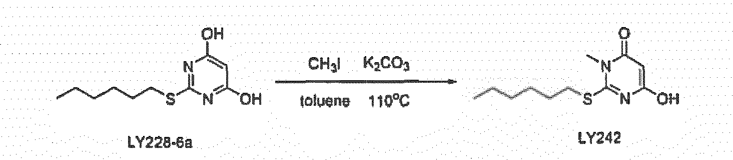

Compound **LY228-6a** (100 mg, 0.44 mmol, 1.0 eq.) was dissolved in toluene, added K₂CO₃ (120 mg, 0.88 mmol, 2.0 eq.), and then, slowly added methyl iodide (62.04 mg, 0.44 mmol, 1.0 eq.) dropwise at 0° C. The reaction was refluxed for 3 h monitored by TLC. After full completion, the mixture was concentrated under vacuum. The crude product was chromatographed (DCM:MeOH=30:1) to yield compound LY242 (white solid, 21 mg, 19.8%). ¹H NMR (300 MHz, DMSO) δ 12.24 (s, 1H), 3.30 (s, 3H), 3.09 (t, *J* = 6.9 Hz, 2H), 1.78 - 1.54 (m, 2H), 1.44 - 1.30 (m, 2H), 1.25-1.28 (m, 4H), 1.01 - 0.71 (t, *J* = 5.4 Hz, 3H).

The following compound was synthesized in the same manner:

| **Cpd#** | **Chemical structure** | **¹H NMR (300 MHz) data** |
|---|---|---|
| **LY242** | 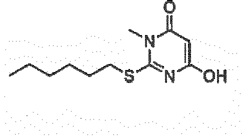 | ¹H NMR (300 MHz, DMSO) δ 12.24 (s, 1H), 3.30 (s, 3H), 3.09 (t, *J* = 6.9 Hz, 2H), 1.78 - 1.54 (m, 2H), 1.44 = 1.30 (m, 2H), 1.25-1.28 (m, 4H), 1.01 - 0.71 (t, *J* = 5.4 Hz, 3H). |
| **LY318** | 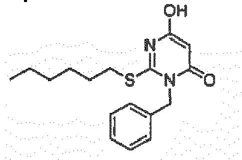 | ¹H NMR (300 MHz, DMSO) δ 7.17 (m, 5H), 3.55 (s, 2H), 3.11 (t, *J* = 7.2 Hz, 2H), 1.60 (m, 2H), 1.36 (m, 2H), 1.26 (m, 4H), 0.86 (t, *J* = 6.3 Hz ,3H). |

### Preparation Example 13 (compound No.: LY238-c)

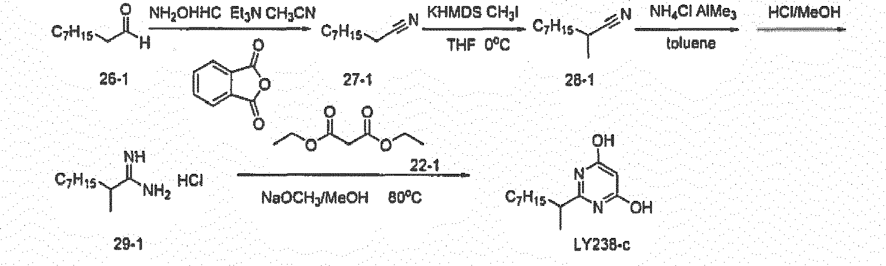

Compound **26-1** (2 mL, 10 mmol, 1 eq.) was dissolved in acetonitrile (20 mL), added hydrazine monohydrochloride (760 mg, 11 mmol, 1.1 eq.) and triethylamine (1.6 mL, 11 mmol, 1.1 eq.) at 0° C. Then, the mixture was stirred at room temperature for 30 minutes, added phthalic anhydride (1.5 g, 10.1 eq, 1.01 eq.), and heated to reflux for 16 h. The mixture was cooled to room temperature and concentrated. The residue was diluted with DCM (10 mL) and filtered to remove the impurities. The filtrate was washed three times with 5% ammonium hydroxide solution (10 mL) and then washed with brine (10 mL). The combined organic layers were concentrated and dried to afford compound **27-1** (yellow oil, 1.35 g, 96%). ¹H NMR (300 MHz, CDCl₃) δ 2.33 (t, *J* = 7.2 Hz, 2H), 1.72 - 1.59 (m, 2H), 1.45 (m, 2H), 1.29 (s, 10H), 0.87 (t, *J* = 6.6 Hz, 3H).

Compound **27-1** (480 mg, 3.46 mmol, 1.0 eq.) was dissolved in THF (10 mL), slowly added dropwise KHMDS (10 mL, 10 mmol, 3 eq.) at 0° C, stirred for 5 minutes, and added methyl iodide (0.42 mL, 3.46 mmol, 2 eq.). After completion as indicated by TLC, the reaction was quenched with water (5mL) and extracted three times with ethyl acetate (5mL). The combined the organic extracts were washed with brine, dried, concentrated, and chromatographed (petroleum ether : ethyl acetate = 30:1) to get compound **28-1** (yellow oil, 150 mg, 28.4%). ¹H NMR (300 MHz, CDCl₃) δ 1.66 - 1.55 (m, 2H), 1.39 (m, 1H), 1.31 (d, *J* = 4.5 Hz, 3H), 1.29 (s, 10H), 0.87 (t, *J* = 6.6 Hz, 3H).

Trimethylaluminum (0.9 mL, 1.67 mmol, 1.7 eq.) was slowly added dropwise to a solution of NH₄Cl (94.4 mg, 1.76 mmol, 1.8 eq.) in anhydrous toluene (10 mL) under N₂ at 0°C. The mixture was stirred at room temperature. Until no methane gas emission, a solution of compound **28-1**(150 mg, 0.98 mmol, 1 eq.) in toluene was slowly added dropwise. The mixture was stirred at 80°C for 15 h. The mixture was cooled to room temperature, added a small amount of silica gel (300 mg), stirred for 10 minutes, and filtered. The filtrate was concentrated, added HCl in MeOH (2 mL, 2N), stirred for 12 h, filtered, and concentrated to give crude product **29-1** (118 mg, yellow solid).

Na (200 mg) was dissolved in methanol (10 mL), added compound **29-1** (118 mg, 0.69 mmol, 1 eq.) and compound **22-1** (70.4 mg, 0.414 mmol, 0.6 eq.). The reaction was heated to reflux for 12 h, then, cooled to room temperature, and concentrated. A small amount of water was added to dissolve the solid, then, the pH of the mixture was adjusted to acidity with hydrochloric acid (IN). A white solid was precipitated, filtered, and chromatographed (DCM: MeOH = 20: 1) to give compound **LY238-c** (white solid, 41 mg, 26.8%). ¹H NMR (300 MHz, DMSO) δ 11.58(s, 2H), 5.06 (s, 1H), 2.62 (m, 1H), 1.63 (m, 2H), 1.32 (m, 2H), 1.19 (m, 8H), 1.15 (d, *J* = 6.9 Hz, 3H), 0.84 (t, *J* = 6.9 Hz, 3H).

### Preparation Example 14 (compound No.: LY225-b)

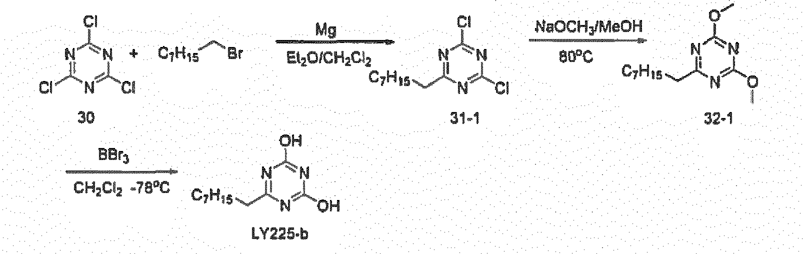

Magnesium (118 mg, 4.92 mmol, 3 eq.) was placed in diethyl ether (10 mL). A small amount of iodine (10 mg) was added, and 1-bromooctane (3 mL, 1.64 mmol, I eq.) was added dropwise. After the initiation, the reaction was heated at 50° C for 1 h and added a solution of compound **30** (300 mg, 1.64 mmol, 1 eq.) in DCM (5 mL), then, stirred at room temperature for 2 h. The reaction mixture turned into orange color. After the reaction was complete monitored by TLC, the reaction was quenched with water (5 mL), extracted three times with DCM (5 mL). The combined the organic extracts were washed with brine and dried. The residue was purified by a flash chromatography on silica gel (petroleum ether : ethyl acetate=30:1) to give compound **31-1.** ¹H NMR (300 MHz, CDCl₃) δ 2.93 - 2.81 (t, *J* = 7.8 Hz, 2H), 1.88 - 1.70 (m, 2H), 1.43 - 1.15 (m, 10H), 0.88 (t, *J* = 6.9 Hz, 3H).

Na (600 mg) was added to methanol (15 mL). After Na was dissolved, compound **31-1** (300 mg, 1.15 mmol, 1 eq.) was added and the mixture was heated to reflux for 16 h. The reaction was monitored by TLC. After the reaction was complete, the reaction mixture was cooled to room temperature and concentrated. The residue was diluted with water (5 mL) and extracted three times with ethyl acetate (5 mL). The organic extracts were combined, washed with brine, and dried. The residue was purified by a flash chromatography on silica gel (DCM:MeOH=50:1) to give compound **32-1** (white solid, 204 mg, 74.7%). ¹H NMR (300 MHz, CDCl₃) δ 4.83 (s, 6H), 2.55 (t, *J* = 7.5 Hz, 2H), 1.81 - 1.65 (m, 2H), 1.33 (m, 10H), 0.88 (t, *J* = 6.9 Hz, 3H).

Compound **32-1** (100 mg, 0.395 mmol, 1 eq.) was dissolved in DCM (5 mL), then slowly added BBr₃ (296 mg, 1.18 mmol, 3 eq.) dropwise at -78°C. The reaction was slowly warmed to 25°C and stirred for 12 h monitored by TLC. After the completion, the reaction was quenched by methanol (3 mL) and concentrated. The residue was purified by a flash chromatography on silica gel (DCM:MeOH=20:1) to give compound **LY225-b** (white solid, 18 mg, 20.2%).¹H NMR (300 MHz, DMSO) δ 11.19 (s, 2H), 2.38 (t, *J* = 7.5 Hz, 2H), 1.59 (m, 2H), 1.25 (m, 10H), 0.84 (t, *J* = 7.5Hz, 3H).

### Preparation Example 15 (compound No.: LY240 & LY224-c)

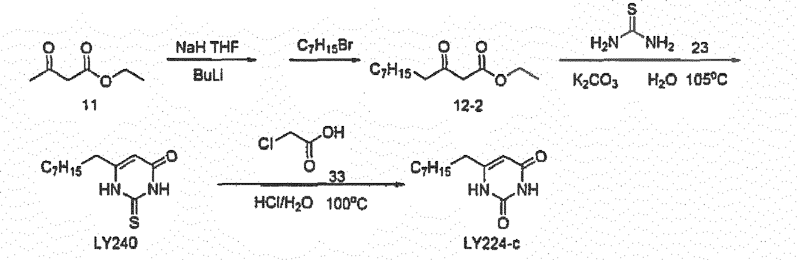

Compound **12-2** was prepared in a manner analogous to compound **12-1;**
Compound **23** (350 mg, 1.54 mmol, 1.5 eq.) was added in water (0.5 mL), stirred at 70°C until compound **23** dissolved, then, K₂CO₃ (213 mg, 1.54 mmol, 1.5 eq.), compound **12-2** (76 mg, 1.0 mmol, I eq.) was added. The reaction was heated at 105°C in an open vessel until the solvent was completely evaporated. The reaction mixture was cooled to room temperature, added water (5mL) to dissolve the solid. The white slurry was obtained. The pH of the mixture was adjusted to acidity with hydrochloric acid (IN). A white sticky solid was formed, and the supernatant was removed. The solid was washed with water (5 mL) for 3 times, and purified by flash chromatography on silica gel (petroleum ether : ethyl acetate = 10:1) to afford compound **LY240** (white solid, 56 mg, 23.3%). ¹H NMR (300 MHz, DMSO) δ 12.30 (s, 1H), 12.19 (s, 1H), 5.67 (s, 1H), 2.33 (t, *J* = 8.1 Hz, 2H), 1.51 (m, 2H), 1.26 (m, 10H), 0.86 (t, *J* = 6.3 Hz, 3H).

Compound 33 (31 mg, 0.42 mmol, 2 eq.) was dissolved in water (1 mL), then slowly added compound LY240 (50 mg, 0.21 mmol, 1 eq.) in THF/H₂O (5/2 mL). The mixture was heated at 70°C for 6 h, and then added concentrated hydrochloric acid (0.1 mL). Thereafter the reaction was carried out at 70°C for 16 h, cooled to room temperature, extracted three times with ethyl acetate (5 mL). The combined organic extracts were washed with brine, dried and concentrated. The residue was purified by flash chromatography on silica gel (DCM:MeOH=20:1) to yield compound **LY224-c** (white solid, 5 mg, 10.6%).¹H NMR (300 MHz, DMSO) δ 10.87 (s, 1H), 10.77 (s, 1H), 5.31 (s, 1H), 2.26 (t, *J* = 7.5 Hz, 2H), 1.51 (m, 2H), 1.25 (m, 10H), 0.86 (t, *J* = 6.6 Hz, 3H).

### Preparation Example 16 (compound No.: LY243)

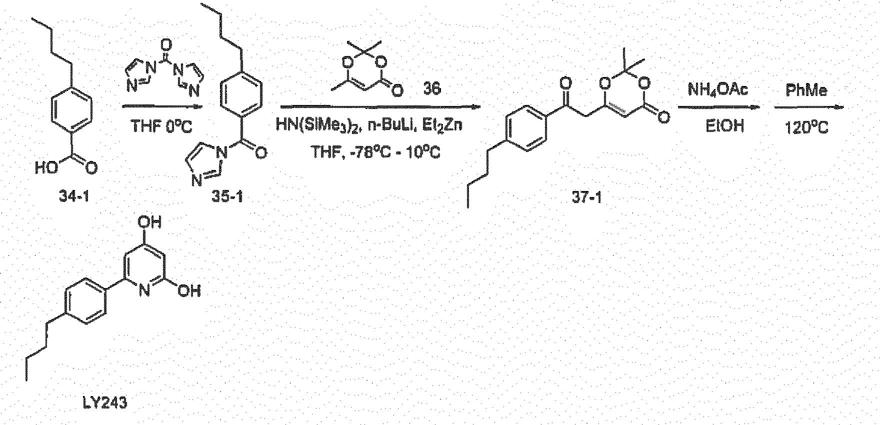

Compound **34-1** (1 g, 5.6 mmol, 1 eq.) was dissolved in THF (15 mL), and then cooled to 0°C, slowly added dropwise 1,1'-carbonyldiimidazole (998 mg, 6.16 mmol, 1.1 eq.) in THF (5 mL) and kept at 0°C for 2 h. The reaction was allowed to warm to 25°C and stirred at 25°C for 1h, then, was added water (5mL), extracted three times with ethyl acetate (10mL). The combined organic extracts were dried over anhydrous sodium sulfate, filtered and concentrated to obtain a crude product 35-1, which are used without further purification.

*n*-BuLi (2.9 mL, 4.61 mmol, 1.4 eq.) was mixed with hexamethylsilane (742 mg, 4.61 mmol, 1.4 eq.) at -78°C, then stirred for 20 min at -78°C, and compound 36 (467 mg, 3.29 mmol, 1.0 eq.) was added, stirred at -78°C for I h, then diethyl zinc (4.6 mL, 4.61 mmol, 1.4 eq.) was added, stirred for 20 min. The mixture was warmed to -20°C, added a solution of compound **35-1** (900 mg, 3.95 mmol) in THF (5 mL). The mixture was warmed to -10°C, stirred for 3 h, quenched with saturated NH₄Cl (10 mL), and extracted with ethyl acetate (10 mL) for 3 times. The combined organic extracts were dried, filtered, concentrated, and purified by flash chromatography on silica gel to afford compound **37-1** (white solid, 321 mg, 27%).

Compound 37-1 (100 mg, 0.33 mmol, 1.0 eq.) was dissolved in ethanol (10 mL), added ammonium acetate (76 mg, 0.99 mmol, 3.0 eq.), and stirred at 25°C for 3 h. The reaction was concentrated, and added toluene (5 mL). The mixture was refluxed at 120°C for 3 h for removing water. The reaction was concentrated and purified by flash chromatography on silica gel (DCM:MeOH=20:1) to give compound **LY243** (yellow solid, 38 mg, 48.7%).¹H NMR (300 MHz, DMSO) δ 11.67 (s, 2H), 7.60 (d, *J* = 8.1 Hz, 2H), 7.18 (d, *J* = 8.1 Hz, 2H), 6.54 (s, 1H), 5.21 (s, 1H), 2.5 (t, *J* = 7.5 Hz, 2H), 1,46-1.38 (m, 2H), 1.32 - 1.00 (m, 2H), 0.75 (t, *J* = 7.5 Hz, 3H).

### Preparation Example 17 (compound No.: LY239)

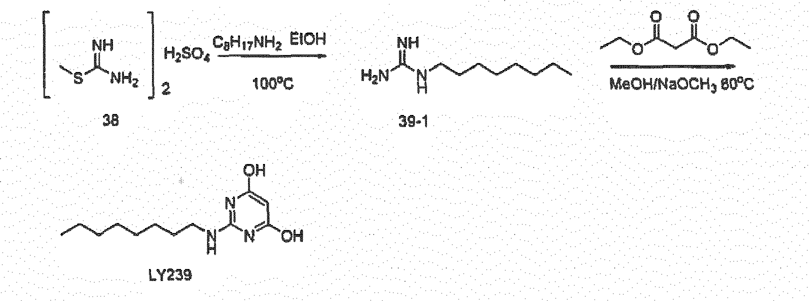

Compound **38** (500 mg, 1.8 mmol, 1.0 eq.) was dissolved in ethanol (10 mL), added octylamine (387 mg, 3 mmol, 1.6 eq.) and heated at 100°C for 16 h. The reaction mixture was concentrated, added to water (5 mL) and ethanol (5 mL). Under ice bath, a white solid was precipitated and filtered to give crude product **39-1** (white solid, 321 mg).

Na (200 mg) was added to methanol (20 mL). After Na was dissolved, compound 39-1 (700 mg, 4.1 mmol, 1 eq.) was added. The mixture was heated to reflux for 16 h, monitored by TLC. After the reaction was complete, the reaction mixture was cooled to room temperature and concentrated, added water (5 mL). The pH of the mixture was adjusted to 3-4 with 1 M hydrochloric acid. The mixture was extracted three times with ethyl acetate (5 mL). The combined organic extracts were washed with brine, dried. The residue was purified by flash chromatography on silica gel (DCM:MeOH=50:1) compound **LY239** (white solid, 132 mg, 13.5%). ¹H NMR (300 MHz, DMSO) δ 10.29 (s, 2H), 6.48 (s, 1H), 4.58 (s, 1H), 3.20 (dd, *J* = 12.9, 6.9 Hz, 2H), 1.46 (m, 2H), 1.26 (m, 10H), 0.85 (t, *J* = 6.9 Hz, 3H).

### Biological Experiment Example

Detection of Cytoplasmic Calcium Ion Concentration with Fluo-4 Fluorescent Dye Tracer Assay

### 1. Purpose

The GPR84 agonist activity of the compounds of the invention was tested.

### 2. Source of material

The human GPR84 cell line was obtained by transfecting a plasmid encoding the GPR84 and G16 proteins in the HEK293 cell line. The fluorescent dye Fluo-4 AM was purchased from Invitrogen.

### 3. Principle

Intracellular Ca²⁺ ion is a very important second messenger of G protein-coupled receptor signaling pathway. When GPR84 coupled to Gα16 protein is bound to a ligand, the concentration of intracellular Ca²⁺ ion can be significantly increased. Fluo-4 is a Ca²⁺ ion-specific fluorescent probe that binds quantitatively to Ca²⁺ ions and emits fluorescence. Therefore, fluorescence assay was used to detect the agonistic activity of compounds in 96-well or 384-well flat bottom microplates. The GPR84 cells were incubated with the Fluo-4 fluorescent dye and added with different concentrations of compounds for stimulation. The changes in the intracellular calcium concentration were detected by the fluorescence intensity of dyes. Fluorescence excitation was 485 nm and the detector for emission was set at 525 nm. Thereby, the concentration for 50% of maximal effect (EC₅₀) was calculated.

### 4. Procedure

1. Preparation of Hank's Balanced Salt Solution (HBSS): The ingredients, 0.4 g/L KCl (5.4 mM), 0.12 g/L Na₂HPO₄·12H₂O (0.3 mM), 0.06 g/L KH₂PO₄(0.4 mM), 0.35 g/L NaHCO₃ (4.2 mM), 0.14 g/L CaCl₂ (1.3 mM), 0.10 g/L MgCl₂·6H₂O (0.5 mM), 0.05 g/L MgSO₄ (0.6 mM), 8.0 g/L NaCl (137 mM), were weighed and dissolved with ultrapure water. The pH of the solution was adjusted to 7.4 with hydrochloric acid or NaOH. The solution was filtered, and stored at 4°C for one month.
2. Preparation of Ca²⁺ buffer: Firstly, a 560 mM D-glucose (100 X) stock solution and a 250 mM Sulfinpyrazone (1000 X) stock solution were prepared. Then, to 100 mL of HBSS, was added BSA (0.5 g), 560 mM D-glucose stock solution (1 mL) and 250 mM Sulfinpyrazone (100 µL). The final concentrations in Ca²⁺ buffer were 0.5% BSA, 5.6 mM D-glucose, 250 µM Sulfinpyrazone. The Ca²⁺ buffer was mixed and used as freshly prepared.
3. Preparation of dyes solution: Firstly, a stock solution of 3% Cremophor EL (100X) in PBS and a stock solution of 2 mM Fluo-4 (1000X) in DMSO was prepared. Secondly, one milliliter of dye solution was prepared by mixing 1 µL of 2 mM Fluo-4 AM with 10 µL of 3% Cremophor EL and diluting with 1 mL of Ca²⁺ buffer and mixed.
4. The GPR84 cells were cultured in a 96-well plate at a starting density of 4 × 10⁴ cells. The cells were continually cultured for more than 24 hours so that the cell density was 80-90% for detection.
5. The culture fluid was removed from the cells to be tested. The cells were added freshly prepared dyes and incubated in a 37-degree incubator for 40 minutes to 50 minutes.
6. Preparation of dyes solution: Compounds are dissolved and diluted to 3-fold the final working concentration with freshly prepared Ca²⁺ buffer. If compounds are dissolved in DMSO, the final DMSO concentration should not exceed 1%.
7. After the incubation completed, the dye was removed. The cells were washed with Ca²⁺ buffer and then incubated with an additional 50 µL of Ca²⁺ buffer for 5 to 10 minutes.
8. The cells were stimulated with 25 µL/well of Ca²⁺ buffer containing different concentrations of the compound. The plate was read by using the FlexStation III Multi-Mode Microplate Reader. The changes in the intracellular calcium concentration were detected by the fluorescence intensity of dyes. Fluorescence excitation was 485 nm and the detector for emission was set at 525 nm.

Taking GPR84 receptor as an example, agonist 6-OAU sampling situation is listed as below:

| 6-OAU initial concn. | 6-OAU sample concn. | 6-OAU final concn. |
|---|---|---|
| 10 mM (with 100%DMSO) | 30 µM (with 3%DMSO) | 100 µM (with 1%DMSO) |
| 1 mM (with 100%DMSO) | 30 µM (with 3%DMSO) | 10 µM (with 1%DMSO) |
| 100 nM (with 100%DMSO) | 3 µM (with 3%DMSO) | 1 µM (with 1%DMSO) |
| 10 µM (with 100%DMSO) | 300 nM (with 3%DMSO) | 100 nM (with 1%DMSO) |
| 1 µM (with 100%DMSO) | 30 nM (with 3%DMSO) | 10 nM (with 1% DMSO) |
| 100 nM (with 100%DMSO) | 3 nM (with3%DMSO) | 1 nM (with 1%DMSO) |
| 10 nM (with 100%DMSO) | 0.3 nM (with 3%DMSO) | 0.1 nM (with 1%DMSO) |
| 100%DMSO | 3%DMSO | 1%DMSO |

### 5. Result:

| **Compound No.** | **EC₅₀(µM)** | **Compound No.** | **EC₅₀(µM)** |
|---|---|---|---|
| **LY214-5** | 2.479 | **LY196** | 8.852 |
| **LY228-6a** | 0.2114 | **LY210** | 21.56 |
| **LY228-6b** | 0.6408 | **LY212** | 43.43 |
| **LY242-7** | 0.2311 | **LY248** | 3.856 |
| **LY256-8** | 0.2639 | **LY262-a** | 0.5728 |
| **LY312-12** | 1.164 | **LY276** | 0.4351 |
| **LY340-14** | 25.69 | **LY266-a** | 2.944 |
| **LY368-16** | 11.31 | **LY266-c** | 2.592 |
| **LY290-a** | 1.076 | **LY224-a** | 0.6531 |
| **LY266-b** | 1.991 | **LY262-b** | 0.271 |
| **LY226** | 0.9236 | **LY196b** | 2.105 |
| **LY182** | 3.669 | **LY210-b** | 0.01274 |
| **LY196** | 0.4542 | **LY238** | 0.04871 |
| **LY224-b** | 0.01135 | **LY244** | 0.08809 |
| **LY236** | 0.4347 | **LY237** | 0.00019 |
| **LY243** | 0.006099 | **LY224-c** | 0.8109 |
| **LY223** | 0.001339 | **LY238-c** | 0.2988 |
| **LY225-b** | 1.023 | **LY240** | 0.3398 |
| **LY238-d** | 0.003511 | **LY209** | 0.001254 |
| **LY239** | 0.00614 | **6-OAU** | 0.661-0.919 |

A series of compounds was proved to have excellent agonistic activity against GPR84. Especially activity of compound **LY237** is 4500 times higher than 6-OAU, which is the best reported agonistic activity at present.

## Claims

1. A compound represented by formula **I** or a pharmaceutically acceptable salt thereof, 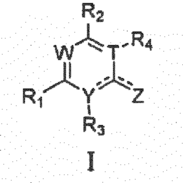 wherein, R₁ is R₁ₐ, R_{1b} or R_{1c}; 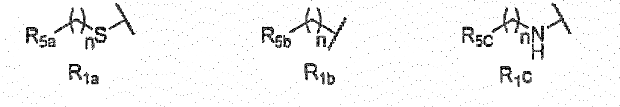
each of R₅ₐ, R_{5b} and R_{5c} is independently methyl, isopropyl, C2-C9 alkenyl, C2-C4 alkynyl, 3-6 membered cycloalkyl, cyano, hydroxy, unsubstituted phenyl, phenyl substituted with C1-C4 alkyl, substituted phenyl substituted with C1-C3 alkoxy, or fluorophenyl;
subscript n is an integer selected from 0-16;
T, W and Y are each independently O, N or C;
R₂ is hydroxy, amino, trifluoromethyl, or C1-C3 alkyl;
R₃ is absent or is hydrogen, benzyl or C1-C6 alkyl;
R₄ is absent or is hydrogen or C1-C3 alkyl;
Z is -OH, -NH₂, =O, =S or C1-C6 alkylcarbonyl.

2. A compound or pharmaceutically acceptable salt thereof according to claim 1, wherein,
R₁ is R₁ₐ, R_{1b} or R_{1c}; 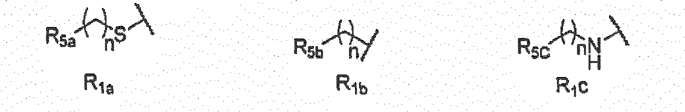
each of R₅ₐ, R_{5b} and R_{5c} is independently methyl, isopropyl, C2-C9 alkenyl, ethynyl, 3-4 membered cycloalkyl, cyano, hydroxy, unsubstituted phenyl, phenyl substituted with C1-C4 alkyl, methoxyphenyl, or fluorophenyl;
subscript n is an integer selected from 0-9;
T, W and Y are each independently N or C;
R₂ is hydroxy, amino, trifluoromethyl, or methyl;
R₃ is absent or is hydrogen or benzyl;
R₄ is absent or is hydrogen or methyl;
Z is -OH, -NH₂, -O, =S, or C1-C6 alkylcarbonyl.

3. A compound or pharmaceutically acceptable salt thereof according to claim 1, wherein,
R₁ is R₁ₐ, wherein R₅ₐ is methyl, isopropyl, 3-4 membered cycloalkyl, unsubstituted phenyl, cyano, hydroxy, phenyl substituted with C1-C4 alkyl, methoxyphenyl, or fluorophenyl;
R₂ is hydroxy;
subscript n is an integer selected from 0-14;
T, W and Y are each independently O, N or C;
R₃ is absent or is hydrogen, benzyl or C1-C3 alkyl;
R₄ is absent or is hydrogen or C1-C3 alkyl;
Z is -OH, -NH₂, =O, =S or C1-C6 alkylcarbonyl.

4. A compound or pharmaceutically acceptable salt thereof according to claim I, wherein the compound of the formula **I** has the structure of Formula **II**: 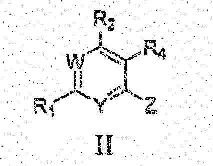
R₁ is R₁ₐ, R_{1b} or R_{1c};
R₂ is hydroxy, methyl, amino, or trifluoromethyl;
W and Y are each independently N;
R₄ is hydrogen or C1-C3 alkyl;
Z is -OH or -NH₂.

5. A compound or pharmaceutically acceptable salt thereof according to claim 1, wherein the compound of the formula **I** has the structure of Formula **III**: 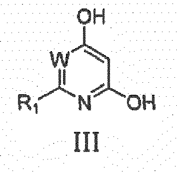 wherein,
R₁ is R₁ₐ, R_{1b} or R_{1c};
W is N or C.

6. A compound or pharmaceutically acceptable salt thereof according to claim 1, wherein, the compound is selected from:

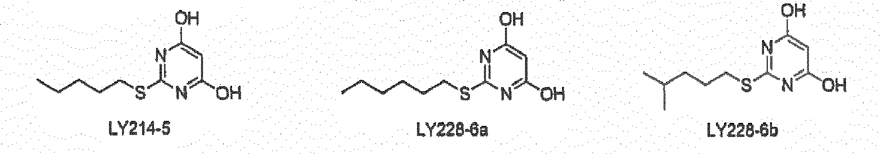

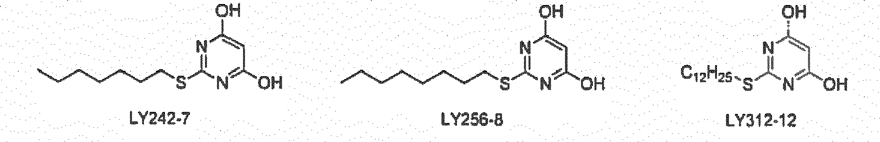

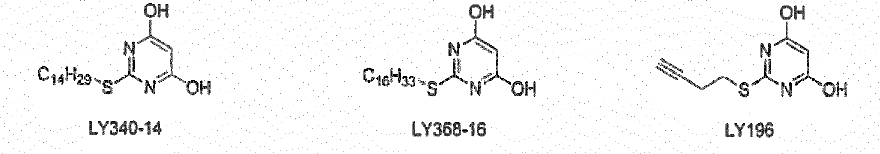

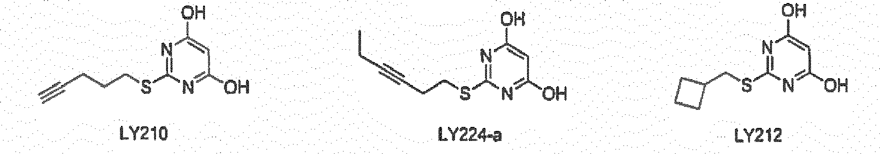

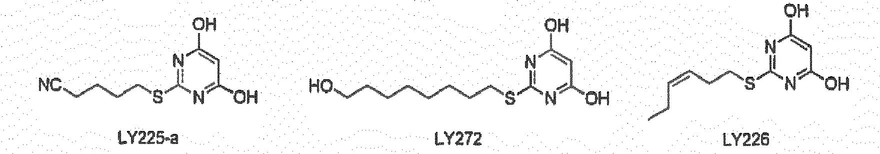

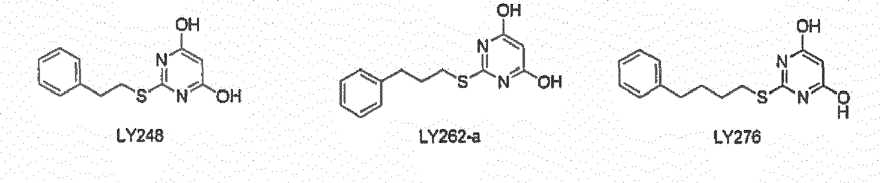

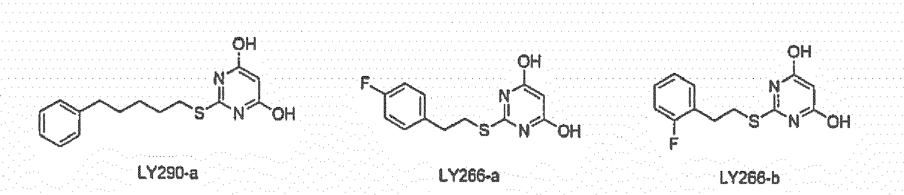

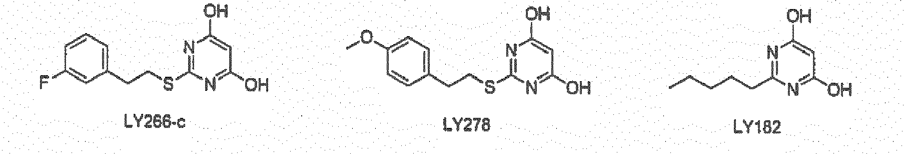

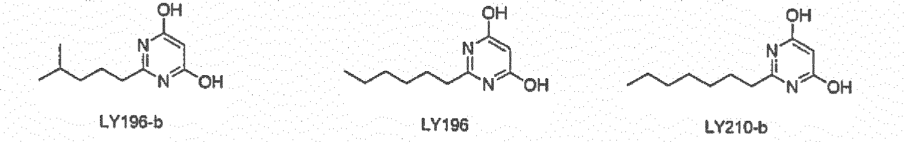

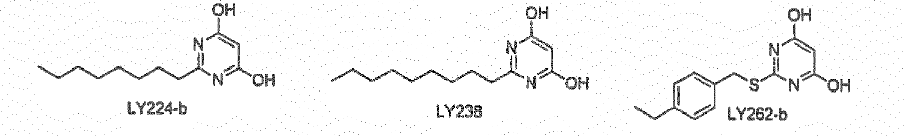

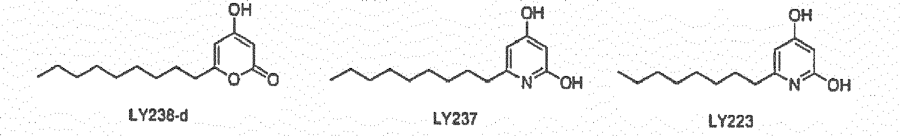

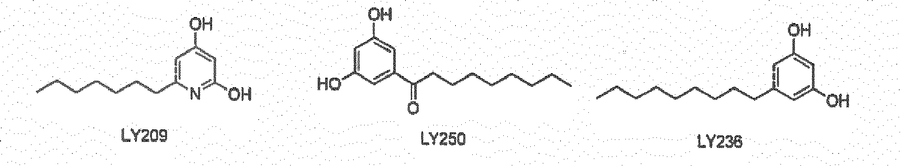

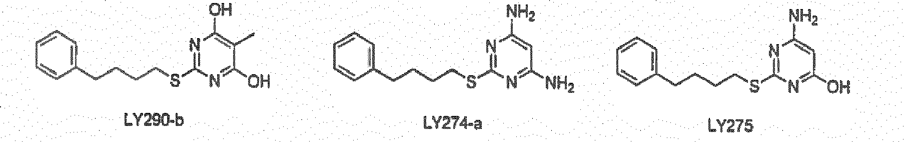

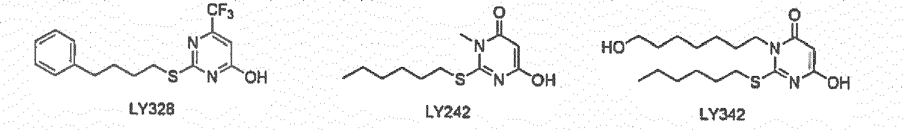

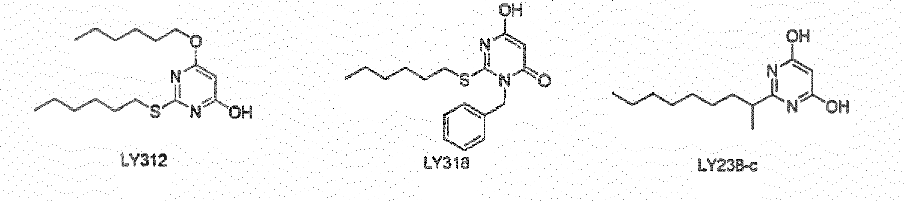

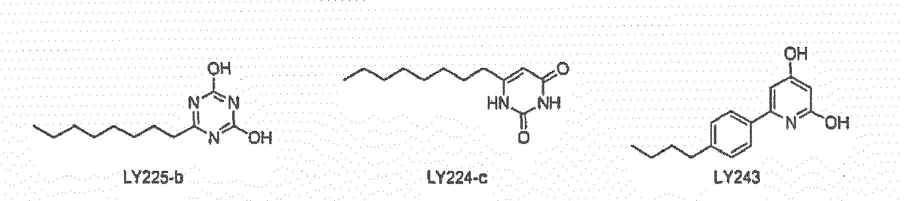

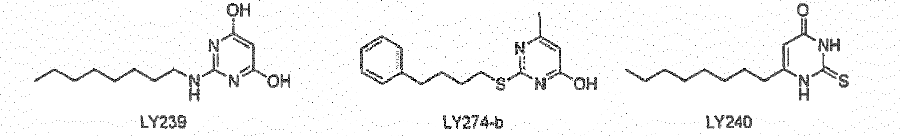

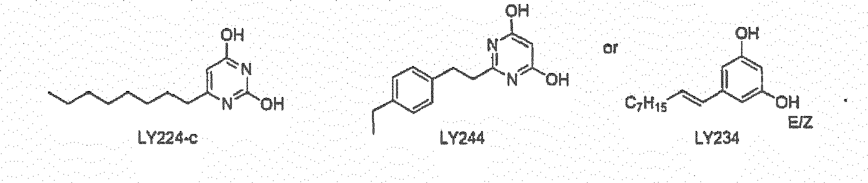

7. A pharmaceutical composition comprising a therapeutically effective amount of one or more selected from the group consisting of the compound or pharmaceutically acceptable salts thereof according to any one of claims 1-6, and one or more pharmaceutically acceptable carriers.

8. A use of the compound or pharmaceutically acceptable salts thereof according to any one of claims 1-6, in preparing a medicament for the treatment of septicemia.
